(19) Europäisches Patentamt
European Patent Office
Office européen des brevets

(11) Publication number: **0 294 892 B1**

(12) # EUROPEAN PATENT SPECIFICATION

(45) Date of publication of patent specification: **18.08.93**

(51) Int. Cl.$^5$: **C11D 1/58**

(21) Application number: **88201147.1**

(22) Date of filing: **06.06.88**

(54) **Conditioning agents and compositions containing same.**

(30) Priority: **10.06.87 US 61061**

(43) Date of publication of application:
**14.12.88 Bulletin 88/50**

(45) Publication of the grant of the patent:
**18.08.93 Bulletin 93/33**

(84) Designated Contracting States:
**AT BE CH DE ES FR GB IT LI LU NL**

(56) References cited:
**EP-A- 0 007 135** **EP-A- 0 268 324**
**FR-A- 2 526 441** **GB-A- 2 195 653**
**US-A- 4 000 077** **US-A- 4 255 484**

(73) Proprietor: **THE PROCTER & GAMBLE COMPANY**
**One Procter & Gamble Plaza**
**Cincinnati Ohio 45202(US)**

(72) Inventor: **Schmidt Baker, Ellen**
**68 Versailles**
**Cincinnati, OH 45240(US)**
Inventor: **Mast, Roy Clark**
**3336 Nandale Dr.**
**Cincinnati, OH 45239(US)**
Inventor: **Hartman, Frederick Anthony**
**10347 Deerfield Rd.**
**Cincinnati, OH 45242(US)**

(74) Representative: **Canonici, Jean-Jacques et al**
**Procter & Gamble European Technical Center N.V. Temselaan 100**
**B-1853 Strombeek-Bever (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

### TECHNICAL FIELD

This invention relates to conditioning agents and more particularly to compositions containing these agents, such as laundry detergents, which impart fabric conditioning benefits through the wash.

### BACKGROUND OF THE INVENTION

Numerous attempts have been made to formulate laundry detergent compositions which provide the good cleaning performance expected of them and which also have good textile softening properties. Attempts have been made to incorporate cationic textile softeners in anionic surfactant-based built detergent compositions employing various means of overcoming the natural antagonism between the anionic and cationic surfactants. For instance, U.S. Patent 3,936,537, Baskerville et al., issued February 3, 1976, discloses detergent compositions comprising organic surfactant, builders, and, in particulate form (10 to 500 μm), a quaternary ammonium softener combined with a poorly water-soluble dispersion inhibitor which inhibits premature dispersion of the cationic in the wash liquor. US-A-4 000 077 and FR-A-2 526 441 discloses mixtures of cationic and anionic compounds as conditioning agents. Even in these compositions some compromise between cleaning and softening effectiveness has to be accepted. Another approach to provide built detergent compositions with softening ability has been to employ nonionic surfactants (instead anionic surfactants) with cationic softeners. Compositions of this type have been described in, for example, German Patent 1,220,956, assigned to Henkel, issued April 4, 1964; and in U.S. Patent 3,607,763, Salmen et al., issued September 21, 1971. However, the detergency benefits of nonionic surfactants are inferior to those of anionic surfactants.

Other laundry detergent compositions have employed tertiary amines along with anionic surfactants to act as textile softeners. British Patent 1,514,276 Kengon, published June 14, 1978, employs certain tertiary amines with two long chain alkyl or alkenyl groups and one short chain alkyl group. These amines are useful as fabric softeners in detergent compositions when their isoelectric point is such that they are present as a dispersion of negatively charged droplets in the normally alkaline wash liquor, and in a more cationic form at the lower pH of a rinse liquor, and so become substantive to fabrics. The use of such amines, among others, in detergent compositions has also been previously disclosed in British Patent 1,286,054, assigned to Colgate-Palmolive, published August 16, 1972.

Another approach to provide anionic detergent compositions with textile softening ability has been the use of smectite-type clays, as described in U.S. Patent 4,062,647, Storm et al., issued December 13, 1977. These compositions, although they clean well, require large contents of clay for effective softening. The use of clay together with a water-insoluble cationic compound in an electrically conductive metal salt as a softening composition adapted for use with anionic, nonionic, zwitterionic and amphoteric surfactants has been described in British Patent 1,483,627, assigned to Procter & Gamble, published August 24, 1977.

Dryer-added fabric conditioning compositions are disclosed in EP-A-7 135 and US-A-4 255 484.

Laundry detergents containing imidazolines have been disclosed before. See, for example, U.S. Patent 4,589,988, Rieck et al., issued May 20, 1986, which discloses granular laundry detergents containing a combination of surfactant, and a softener system comprising amine or imidazoline and a phyllosilicate. The amine or imidazoline component is adsorbed onto the clay silicate particles. U.S. Patent 4,294,710, Hardy, et al., issued October 13, 1981, discloses granular laundry detergents containing a combination of surfactants along with tertiary amines or imidazoline derivatives. Generally, such detergent compositions are prepared such that the amine is sprayed onto the particulate detergent components. This reference does not recognize the criticality of particle size of the imidazoline for imparting fabric care benefits.

British Patent Applications 1,077,103 and 1,077,104, assigned to Bayer, published July 26, 1967, disclose amine-anionic surfactant ion-pair complexes useful as antistatic agents. These complexes are applied directly to the fabric from an aqueous carrier. There is no suggestion in either of these references that such complexes could be added to detergent compositions to impart fabric care benefits through-the-wash. In fact, such complexes are delivered in solubilized form and therefore could not be delivered through-the-wash.

Fatty acid-amine ion-pair complexes in granular detergents are disclosed in European Patent Application 133,804, Burckett-St.Laurent et al., published June 3, 1985. While this complex delivers fabric conditioning benefits, the imidazoline-anionic surfactant ion-pair complexes of the present invention provide superior antistatic performance.

Surprisingly, it has been found that imidazoline-anionic surfactant ion-pair complex particles are excellent conditioning agents when applied alone or in certain laundry detergent compositions containing these imidazoline-anionic surfactant ion-pair complexes.

It is therefore an object of the present invention to provide certain imidazoline-anionic surfactant ion-pair complexes with an average particle size diameter of from 10 to 300 microns, and compositions containing these particles, which provide excellent conditioning benefits.

## SUMMARY OF THE INVENTION

The present invention relates to conditioning agents comprising water-insoluble particles having an average diameter of from 10 to 300 microns, comprising an imidazoline-anionic surfactant ion-pair complex with a ratio of imidazoline to anionic surfactant of from 10:1 to 1:1 having the formula:

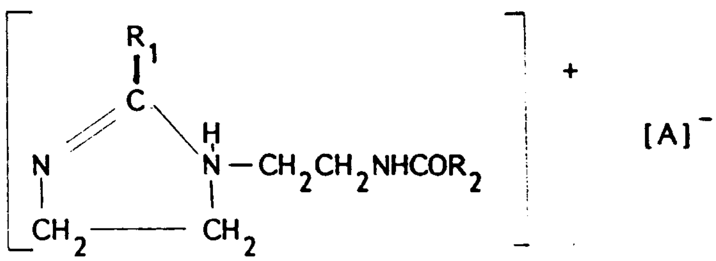

wherein $R_1$ and $R_2$ can independently be $C_{12}$ to $C_{20}$ hydrocarbon, and A is an anionic surfactant selected from the group consisting of aryl sulfonates, alkyl aryl sulfonates, paraffin sulfonates, alkyl ethoxylated sulfates, olefin sulfonates, dialkyl sulfosuccinates, ethoxylated alkyl sulfonates, alkyloxybenzene sulfonates, acyl isethionates and acylalkyl taurates and mixtures of such ion-pair complexes. Preferred imidazolines include those where $R_1$ and $R_2$ are independently chosen from $C_{14}$ to $C_{20}$ alkane or alkene, more preferably $C_{16}$ to $C_{20}$ alkane and even more preferably $C_{16}$ to $C_{18}$ alkane.

When incorporated into a detergent composition, the agents comprise from 0.1 to 25% by weight of the composition. The preferred detergent surfactants are anionic surfactants. These detergent compositions can optionally contain clay softening materials, detergent builders, chelating agents and/or peroxygen bleaching agents.

## DETAILED DESCRIPTION OF THE INVENTION

The components of the present invention are described in detail below.

### Conditioning Agent

The conditioning agent of the present invention consists of water-insoluble particles having an average diameter of from 10 to 300, preferably from 10 to 250, more preferably from 10 to 200, more preferably from 10 to 150 $\mu$m, and most preferably 40 to 110 $\mu$m, said particles comprising an imidazoline-anionic surfactant ion-pair complex.

Preferred imidazoline-derivatives are those wherein $R_1$ and $R_2$ are independently $C_{12}$ to $C_{20}$ alkane and alkene, and more preferably $C_{14}$ to $C_{20}$ alkane. Suitable examples of such imidazoline derivatives include stearylamidoethyl-2-stearyl imidazoline, stearylamidoethyl-2-palmityl imidazoline, stearylamidoethyl-2-myristyl imidazoline, palmitylamidoethyl-2-palmityl imidazoline, palmitylamidoethyl-2-myristyl imidazoline, stearylamidoethyl-2-tallow imidazoline, myristylamidoethyl-2-tallow imidazoline, palmitylamidoethyl-2-tallow imidazoline, coconut-amidoethyl-2-coconut imidazoline, tallowamidoethyl-2-tallow imidazoline and mixtures of such imidazoline derivatives. More preferred are those imidazoline derivatives wherein $R_1$ and $R_2$ are independently $C_{16}$ to $C_{20}$ alkane (e.g. wherein $R_1$ and $R_2$ are derived from palmityl, stearyl and arachidyl). Most preferred are those imidazoline derivatives wherein $R_1$ and $R_2$ are independently $C_{16}$ to $C_{18}$ alkyl, i.e., wherein $R_1$ and $R_2$ are each derived from hydrogenated tallow.

These imidazoline derivatives can be manufactured, for example, from the reaction of diethylene triamine with the appropriate carboxylic acid. This procedure is set forth in Kirk-Othmer Encyclopedia of Chemical Technology, Third Edition, Volume 7, pages 580-600 (Grayson et al., Editors; Wiley-Interscience, N.Y., N.Y; 1979).

Preferred $C_{16}$ to $C_{18}$ imidazoline derivatives are available from Sherex Corporation as Varisoft® 445 imidazoline. Varisoft® 445 imidazoline may contain up to 50% of non-imidazoline material (e.g., starting materials) which do not adversely affect the conditioning benefits of the present invention.

The anionic surfactants (A) useful in the ion-pair complex of the present invention are the aryl sulfonates, alkylaryl sulfonates, paraffin sulfonates, olefin sulfonates, alkyl ethoxylated sulfates, dialkyl sulfosuccinates, ethoxylated alkyl sulfonates, alkyl oxybenzene sulfonates, acyl isethionates and acylalkyl taurates. These classes of anionic surfactants are fully described herein.

More preferred are the alkyl ethoxylated sulfates, linear $C_1$ to $C_{20}$ alkylaryl sulfonates, aryl sulfonates and sulfosuccinates.

Particularly preferred are the linear $C_1$ to $C_{20}$ alkylaryl sulfonates and more particularly preferred are the linear $C_1$-$C_{13}$ alkylaryl sulfonates. This class of surfactants includes the linear $C_1$-$C_{13}$ alkyl benzene sulfonates which are particularly preferred for use herein.

Non-limiting examples of ion-pair complexes suitable for use in the present invention include:
stearylamidoethyl-2-stearyl imidazoline complexed with a $C_1$-$C_{20}$ linear alkylbenzene sulfonate (LAS),
stearylamidoethyl-2-palmityl imidazoline complexed with a $C_1$-$C_{20}$ LAS,
stearylamidoethyl-2-myristyl imidazoline complexed with a $C_1$-$C_{20}$ LAS,
palmitylamidoethyl-2-palmityl imidazoline complexed with a $C_1$-$C_{20}$ LAS,
palmitylamidoethyl-2-myristyl imidazoline complexed with a $C_1$-$C_{20}$ LAS,
stearylamidoethyl-2-tallow imidazoline complexed with a $C_1$-$C_{20}$ LAS,
myristylamidoethyl-2-tallow imidazoline complexed with a $C_1$-$C_{20}$ LAS,
palmitylamidoethyl-2-tallow imidazoline complexed with a $C_1$-$C_{20}$ LAS,
coconut-amidoethyl-2-coconut imidazoline complexed with a $C_1$-$C_{20}$ LAS,
hydrogenated tallowamidoethyl-2-hydrogenated tallow imidazoline complexed with a $C_1$-$C_{20}$ LAS.

The imidazoline and surfactant components are combined in a molar ratio of imidazoline to surfactant ranging from 10:1 to 1:1, preferably from 8:1 to 1:1, more preferably from 5:1 to 2:1 and most preferably about 4:1. This can be accomplished by any of a variety of means, including but not limited to, preparing a melt of the anionic surfactant (in acid form) and the imidazoline, preferably under a nitrogen blanket, and then processing to the desired particle size range.

Other methods of forming the ion-pair complex include dissolving the components in an organic solvent, or by heating the imidazoline to a liquid state and then adding this molten imidazoline component to a heated acidified aqueous solution of the anionic surfactant, and then extracting the ion-pair complex by using a solvent, such as chloroform.

The complexing of the imidazoline and the anionic surfactant results in an entity (ion-pair) which is chemically distinct from either of the two starting materials.

It has been found that in order for these imidazoline-anionic surfactant ion-pair complexes to impart their conditioning benefits they must have an average particle diameter of from 10 to 300 µm, preferably from 10 to 250 µm, more preferably from 10 to 200 µm, more preferably from 10 to 150 µm, and most preferably from 40 µm to 110 µm. The term "average particle diameter" represents the mean particle size diameter of the actual particles of a given material. The mean is calculated on a weight percent basis. The mean is determined by conventional analytical techniques such as, for example, laser light diffraction or microscopic determination utilizing a scanning electron microscope. Preferably, greater than 50% by weight and more preferably greater than 60% by weight and most preferably greater than 70% by weight, of the particles have actual diameters which fall within the range of from 10 to 300 µm, preferably from 10 to 250 µm, more preferably from 10 to 200 µm, more preferably from 10 to 150 µm and most preferably from 40 µm to 110 µm.

The desired particle sizes can be achieved by, for example, mechanically grinding the resulting ion-pair complex in blenders (e.g., an Oster® blender) or in large scale mills (e.g., a Wiley® Mill) to the desired particle size range or by prilling in a conventional manner (e.g., forcing the well-circulated comelt through a heated nozzle into cooled atmospheric temperatures).

Such factors as the type of imidazoline and type of anionic surfactant employed and the ratio of the imidazoline-surfactant components can affect the physical properties of the resulting complex. Complexes which are gelatinous at room temperature can be mechanically ground to achieve the desired particle size after flash freezing by using, for example, liquid nitrogen. For laundry applications, when less than 5% of the particles have an average diameter of above 110 microns, problems with laundry appliance compatibility can be avoided. However, when complexes with melting points below 75 °C are used, a somewhat higher percentage of larger particles can be added.

These ion-pair complexes can also be used for imparting conditioning themselves or from a variety of carriers. For example, in a laundry application, these complexes can be added during the wash or rinse

cycle. They can also be added along with other fabric conditioners such as smectite clays. Other compositions for use of these agents include detergent compositions (both granular and liquid), shampoo compositions, and washer-added and dryer-added fabric conditioners. These compositions are fully described in the following issued U.S. Patents 4,103,047, Zaki et al., issued July 25, 1978 and 4,233,164, Davis, issued November 11, 1980. These conditioning agents and/or laundry actives can also be adapted for use in a dissolvable laundry product, such as a dissolvable pouch.

A fiber or fabric conditioner of the present invention comprises the imidazoline-anionic surfactant ion-pair complexes of the present invention along with a liquid carrier. Compositions of this invention can also be adapted to a thru-the-wash, compact, laminated laundry product which comprises powdered laundry actives laminated between two plies, at least one ply of which is a strong wet-strength, high stretch paper tissue having one or a multiplicity of deeply embossed (stretched) non-connecting tissue cup-like depressions containing the powder active with the other ply covering the cups. The plies are sealed with a glue pattern around the cup rims. The high stretch paper is made to withstand an embossed stretch to form said deeply embossed (stretched) cups and to survive the rigors of a washing machine. This form of thru-the-wash laundry product is fully described in U.S. Patent 4,638,907 to Bedenk et al., issued January 27, 1987. This pouch can contain the conditioning active alone, or in combination with surfactants and other laundry actives (e.g., smectite clay for textile softening).

In order to incorporate these ion-pair complexes into granular detergents, it is preferred that the individual particles be agglomerated using any of a variety of binding agents known in the art in order to form granular-sized (e.g., 1 millimeter) particles. Such binding agents must dissolve quickly in the wash liquor. Suitable examples of binding agents include water, or water-soluble salts such as sulfates, carbonates, Dextrin glue, or phosphates. When these particles are agglomerated prior to their addition to the detergent granules, it minimizes segregation of the particles from the remainder of the detergent composition.

It has been found that these conditioning agents, unlike those of the prior art, can be incorporated into the detergent compositions of the present invention with little, if any, detrimental effect on cleaning. These detergent compositions provide fabric care benefits across a variety of laundry compositions. That is, machine or hand washing and machine drying and also machine or hand washing and line drying. Additionally, these same conditioning agents can be used with a variety of surfactant systems. Such surfactant systems include mixtures of all types of surfactants, i.e., anionics, cationics, nonionics, zwitterionics and amphoterics. Additionally, these conditioning agents can be used with mixtures of surfactants that are within the same class, e.g., two different anionic surfactants. In fact, it has been found that mixed anionic surfactant systems are preferred for use in the present invention. Examples of such mixed anionic surfactant systems include linear $C_9$-$C_{15}$ alkyl benzene sulfonates and $C_{10}$-$C_{20}$ alkyl sulfate.

The detergent compositions of the present invention contain from 0.1% to 25%, preferably from 1% to 10%, most preferably from 4% to 8% of the ion-pair complex component by weight of the total composition.

## Detergent Surfactant

The amount of detergent surfactant included in the compositions of the present invention can vary from 1% to 95% by weight of the composition, depending upon the particular surfactant(s) used and the effects desired. Preferably, the detergents surfactant(s) comprises from 10% to 60% by weight of the composition. Preferred are anionic surfactants, cationic surfactants and nonionic surfactants and mixtures thereof. Anionic surfactants are much preferred for optimum combined cleaning and textile softening performance, but other classes of surfactants such as nonionic, ampholytic, zwitterionic, or cationic may be used. Mixtures of these surfactants can also be used.

### A. Anionic Surfactants

Anionic surfactants suitable for use in the present invention are generally disclosed in U.S. Patent 3,929,678, Laughlin et al., issued December 30, 1975, at column 23, line 58 through column 29, line 23 and in U.S. Patent 4,294,710, Hardy et al., issued October 13, 1981. Classes of useful anionic surfactants include:

1. Ordinary alkali metal soaps, such as the sodium, potassium, ammonium and alkylolammonium salts of higher fatty acids containing from 8 to 24 carbon atoms, preferably from 10 to 20 carbon atoms. Preferred alkali metal soaps are sodium laurate, sodium stearate, sodium oleate and potassium palmitate.

2. Water-soluble salts, preferably the alkali metal, ammonium and alkylolammonium salts, of organic sulfuric reaction products having in their molecular structure an alkyl group containing from 10 to 20 carbon atoms and a sulfonic acid or sulfuric acid ester group. (Included in the term "alkyl" is the alkyl portion of acyl groups).

Examples of this group of anionic surfactants are the sodium and potassium alkyl sulfates, especially those obtained by sulfating the higher alcohols ($C_8$-$C_{18}$ carbon atoms) such as those produced by reducing the glycerides of tallow or coconut oil; and the sodium and potassium alkylbenzene sulfonates in which the alkyl group contains from 9 to 15 carbon atoms, in straight chain or branched chain configuration, e.g., those of the type described in U.S. Patent 2,220,099, Guenther et al., issued November 5, 1940, and U.S. Patent 2,477,383, Lewis, issued December 26, 1946. Especially useful are linear straight chain alkylbenzene sulfonates in which the average number of carbon atoms in the alkyl group is from 11 to 13, abbreviated as $C_{11}$-$C_{13}$LAS.

Another group of preferred anionic surfactants of this type are the alkyl polyethoxylate sulfates, particularly those in which the alkyl group contains from 10 to 22, preferably from 12 to 18 carbon atoms, and wherein the polyethoxylate chain contains from 1 to 15 ethoxylate moieties, preferably from 1 to 3 ethoxylate moieties.

Other anionic surfactants of this type include sodium alkyl glyceryl ether sulfonates, especially those ethers of higher alcohols derived from tallow and coconut oil; sodium coconut oil fatty acid monoglyceride sulfonates and sulfates; and sodium or potassium salts of alkyl phenol ethylene oxide ether sulfonates containing from 1 to 10 units of ethylene oxide per molecule and wherein the alkyl groups contain from 8 to 12 carbon atoms.

Also included are water-soluble salts of esters of alpha-sulfonated fatty acids containing from 6 to 20 carbon atoms in the fatty acid group and from 1 to 10 carbon atoms in the ester group; water-soluble salts of 2-acyloxyalkane-1-sulfonic acids containing from 2 to 9 carbon atoms in the acyl group and from 9 to 23 carbon atoms in the alkane moiety; alkyl ether sulfates containing from 10 to 20 carbon atoms in the alkyl group and from 1 to 30 moles of ethylene oxide; water-soluble salts of olefin sulfonates containing from 12 to 24 carbon atoms; and beta-alkyloxy alkane sulfonates containing from 1 to 3 carbon atoms in the alkyl group and from 8 to 20 carbon atoms in the alkane moiety.

Particularly preferred surfactants for use herein are the linear $C_{11}$-$C_{13}$ alkyl benzene sulfonates and the $C_8$-$C_{18}$ alkyl sulfates and mixtures thereof. Most preferred are mixtures of these two anionic surfactants in a weight ratio of linear alkyl benzene sulfonate to alkyl sulfate is from 0.5:1 to 3:1 and more preferably from 0.5:1 to 2:1.

3. Anionic phosphate surfactants.

4. N-alkyl substituted succinamates.

B. Nonionic Surfactants

Suitable nonionic surfactants are generally disclosed in U.S. Patent 3,929,678, Laughlin et al., issued December 30, 1975, at column 13, line 14 through column 16, line 6. Classes of useful nonionic surfactants include:

1. The polyethylene oxide condensates of alkyl phenols. These compounds include the condensation products of alkyl phenols having an alkyl group containing from 6 to 12 carbon atoms in either a straight chain or branched chain configuration with ethylene oxide, the ethylene oxide being present in an amount equal to from 5 to 25 moles of ethylene oxide per mole of alkyl phenol. Examples of compounds of this type include nonyl phenol condensed with about 9.5 moles of ethylene oxide per mole of phenol; dodecyl phenol condensed with about 12 moles of ethylene oxide per mole of phenol; dinonyl phenol condensed with about 15 moles of ethylene oxide per mole of phenol; and diisooctyl phenol condensed with about 15 moles of ethylene oxide per mole of phenol. Commercially available nonionic surfactants of this type include Igepal® CO-630, marketed by the GAF Corporation; and Triton® X-45, X-114, X-100, and X-102, all marketed by the Rohm & Haas Company.

2. The condensation products of aliphatic alcohols with from 1 to 25 moles of ethylene oxide. The alkyl chain of the aliphatic alcohol can either be straight or branched, primary or secondary, and generally contains from 8 to 22 carbon atoms. Particularly preferred are the condensation products of alcohols having an alkyl group containing from 10 to 20 carbon atoms with from 4 to 10 moles of ethylene oxide per mole of alcohol. Examples of such ethoxylated alcohols include the condensation product of myristyl alcohol with about 10 moles of ethylene oxide per mole of alcohol; and the condensation product of coconut alcohol (a mixture of fatty alcohols with alkyl chains varying in length from 10 to 14 carbon atoms) with about 9 moles of ethylene oxide. Examples of commercially available nonionic surfactants of

this type include Tergitol® 15-S-9 (the condensation product of $C_{11}$-$C_{15}$ linear alcohol with 9 moles ethylene oxide), Tergitol® 24-L-6 NMW (the condensation product of $C_{12}$-$C_{14}$ primary alcohol with 6 moles ethylene oxide with a narrow molecular weight distribution), both marketed by Union Carbide Corporation; Neodol® 45-9 (the condensation product of $C_{14}$-$C_{15}$ linear alcohol with 9 moles of ethylene oxide), Neodol® 23-6.5 (the condensation product of $C_{12}$-$C_{13}$ linear alcohol with 6.5 moles of ethylene oxide), Neodol® 45-7 (the condensation product of $C_{14}$-$C_{15}$ linear alcohol with 7 moles of ethylene oxide), Neodol® 45-4 (the condensation product of $C_{14}$-$C_{15}$ linear alcohol with 4 moles of ethylene oxide), marketed by Shell Chemical Company, and Kyro® EOB (the condensation product of $C_{13}$-$C_{15}$ alcohol with 9 moles ethylene oxide), marketed by The Procter & Gamble Company.

3. The condensation products of ethylene oxide with a hydrophobic base formed by the condensation of propylene oxide with propylene glycol. The hydrophobic portion of these compounds has a molecular weight of from 1500 to 1800 and exhibits water insolubility. The addition of polyoxyethylene moieties to this hydrophobic portion tends to increase the water solubility of the molecule as a whole, and the liquid character of the product is retained up to the point where the polyoxyethylene content is about 50% of the total weight of the condensation product, which corresponds to condensation with up to about 40 moles of ethylene oxide. Examples of compounds of this type include certain of the commercially-available Pluronic® surfactants, marketed by Wyandotte Chemical Corporation.

4. The condensation products of ethylene oxide with the product resulting from the reaction of propylene oxide and ethylenediamine. The hydrophobic moiety of these products consists of the reaction product of ethylenediamine and excess propylene oxide, and generally has a molecular weight of from 2500 to 3000. This hydrophobic moiety is condensed with ethylene oxide to the extent that the condensation product contains from 40% to 80% by weight of polyoxyethylene and has a molecular weight of from 5,000 to 11,000. Examples of this type of nonionic surfactant include certain of the commercially available Tetronic® compounds, marketed by Wyandotte Chemical Corporation.

5. Semi-polar nonionic surfactants which include water-soluble amine oxides containing one alkyl moiety of from 10 to 18 carbon atoms and 2 moieties selected from the group consisting of alkyl groups and hydroxyalkyl groups containing from 1 to 3 carbon atoms; water-soluble phosphine oxides containing one alkyl moiety of from 10 to 18 carbon atoms and 2 moieties selected from the group consisting of alkyl groups and hydroxyalkyl groups containing from 1 to 3 carbon atoms; and water-soluble sulfoxides containing one alkyl moiety of from 10 to 18 carbon atoms and a moiety selected from the group consisting of alkyl and hydroxyalkyl moieties of from 1 to 3 carbon atoms.

Preferred semi-polar nonionic detergent surfactants are the amine oxide surfactants having the formula

$$R^3(OR^4)_x\overset{\overset{O}{\uparrow}}{N}(R^5)_2$$

wherein $R^3$ is an alkyl, hydroxyalkyl, or alkyl phenyl group or mixtures thereof containing from 8 to 22 carbon atoms; $R^4$ is an alkylene or hydroxyalkylene group containing from 2 to 3 carbon atoms or mixtures thereof; x is from 0 to 3; and each $R^5$ is an alkyl or hydroxyalkyl group containing from 1 to 3 carbon atoms or a polyethylene oxide group containing from 1 to 3 ethylene oxide groups. The $R^5$ groups can be attached to each other, e.g., through an oxygen or nitrogen atom, to form a ring structure.

Preferred amine oxide surfactants are $C_{10}$-$C_{18}$ alkyl dimethyl amine oxides and $C_8$-$C_{12}$ alkoxy ethyl dihydroxy ethyl amine oxides.

6. Alkylpolysaccharides disclosed in U.S. Patent 4,565,647, Llenado, issued January 21, 1986, having a hydrophobic group containing from about 6 to about 30 carbon atoms, preferably from 10 to 16 carbon atoms and a polysaccharide, e.g., a polyglycoside, hydrophilic group containing from 1.5 to 10, preferably from 1.5 to 3, most preferably from 1.6 to 2.7 saccharide units. Any reducing saccharide containing 5 or 6 atoms can be used, e.g., glucose, galactose and galactosyl moieties can be substituted for the glucosyl moieties. (Optionally the hydrophobic group is attached at the 2-, 3-, 4-, etc. positions thus giving a glucose or galactose as opposed to a glucoside or galactoside). The intersaccharide bonds can be, e.g., between the one position of the additional saccharide units and the 2-, 3-, 4-, and/or 6-positions on the preceding saccharide units.

Optionally, and less desirably, there can be a polyalkyleneoxide chain joining the hydrophobic moiety and the polysaccharide moiety. The preferred alkyleneoxide is ethylene oxide. Typical hydrophobic groups include alkyl groups, either saturated or unsaturated, branched or unbranched containing from

8 to 18, preferably from 10 to 16, carbon atoms. Preferably, the alkyl group is a straight chain saturated alkyl group. The alkyl group can contain up to 3 hydroxy groups and/or the polyalkyleneoxide chain can contain up to 10, preferably less than 5, alkyleneoxide moieties. Suitable alkyl polysaccharides are octyl, nonyldecyl, undecyldodecyl, tridecyl, tetradecyl, pentadecyl, hexadecyl, heptadecyl, and octadecyl, di-, tri-, tetra-, penta-, and hexaglucosides, galactosides, lactosides, glucoses, fructosides, fructoses and/or galactoses. Suitable mixtures include coconut alkyl, di-, tri-, tetra-, and pentaglucosides and tallow alkyl tetra-, penta-, and hexaglucosides.

The preferred alkylpolyglycosides have the formula

$R^2O(C_nH_{2n}O)_t(glycosyl)_x$

wherein $R^2$ is selected from the group consisting of alkyl, alkylphenyl, hydroxyalkyl, hydroxyalkylphenyl, and mixtures thereof in which the alkyl groups contain from 10 to 18, preferably from 12 to 14, carbon atoms; n is 2 or 3, preferably 2; t is from 0 to 10, preferably 0; and x is from 1.3 to 10, preferably from 1.3 to 3, most preferably from 1.3 to 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4- and/or 6-position, preferably predominantly the 2-position.

7. Fatty acid amine surfactants having the formula:

$$R^6 - \overset{O}{\overset{\|}{C}} - N(R^7)_2$$

wherein $R^6$ is an alkyl group containing from 7 to 21 (preferably from 9 to 17) carbon atoms and each $R^7$ is selected from the group consisting of hydrogen, $C_1$-$C_4$, alkyl, $C_1$-$C_4$ hydroxyalkyl, and $-(C_2H_4O)_xH$ where x varies from 1 to 3.

Preferred amides are $C_8$-$C_{20}$ ammonia amides, monoethanolamides, diethanolamides, and isopropanolamides.

C. Ampholytic Surfactants

Ampholytic surfactants can be broadly described as aliphatic derivatives of secondary or tertiary amines, or aliphatic derivatives of heterocyclic secondary and tertiary amines in which the aliphatic radical can be straight or branched chain and wherein one of the aliphatic substituents contains from 8 to 18 carbon atoms and at least one of the aliphatic substituents contains an anionic water-solubilizing group, e.g., carboxy, sulfonate, sulfate. See U.S. Patent 3,929,678, Laughlin et al., issued December 30, 1975, column 19, line 38 through column 22, line 48, for examples of ampholytic surfactants useful herein.

D. Zwitterionic Surfactants

Zwitterionic surfactants can be broadly described as derivatives of secondary and tertiary amines, derivatives of heterocyclic secondary and tertiary amines, or derivatives of quaternary ammonium, quaternary phosphonium or tertiary sulfonium compounds. See U.S. Patent 3,929,678, Laughlin et al., issued December 30, 1975,column 19, line 38 through column 22, line 48, for examples of zwitterionic surfactants useful herein.

E. Cationic Surfactants

Cationic surfactants can also be included in detergent compositions of the present invention. Cationic surfactants comprise a wide variety of compounds characterized by one or more organic hydrophobic groups in the cation and generally by a quaternary nitrogen associated with an acid radical. Pentavalent nitrogen ring compounds are also considered quaternary nitrogen compounds. Suitable anions are halides, methyl sulfate and hydroxide. Tertiary amines can have characteristics similar to cationic surfactants at washing solutions pH values less than 8.5.

Suitable cationic surfactants include the quaternary ammonium surfactants having the formula:

$$[R^2(OR^3)_y][R^4(OR^3)_y]_2R^5N^+X^-$$

wherein $R^2$ is an alkyl or alkyl benzyl group having from 8 to 18 carbon atoms in the alkyl chain; each $R^3$ is independently selected from the group consisting of $-CH_2CH_2-$, $-CH_2CH(CH_3)-$, $-CH_2CH(CH_2OH)-$, and $-CH_2CH_2CH_2-$; each $R^4$ is independently selected from the group consisting of $C_1$-$C_4$ alkyl, $C_1$-$C_4$ hydroxyalkyl, benzyl, ring structures formed by joining the two $R^4$ groups, $-CH_2CHOHCHOHCOR^6CHOHCH_2OH$ wherein $R^6$ is any hexose or hexose polymer having a molecular weight less than 1000, and hydrogen when y is not 0; $R^5$ is the same as $R^4$ or is an alkyl chain wherein the total number of carbon atoms of $R^2$ plus $R^5$ is not more than 18; each y is from 0 to 10 and the sum of the y values is from 0 to 15; and X is any compatible anion.

Preferred examples of the above compounds are the alkyl quaternary ammonium surfactants, especially the mono-long chain alkyl surfactants described in the above formula when $R^5$ is selected from the same groups as $R^4$. The most preferred quaternary ammonium surfactants are the chloride, bromide and methylsulfate $C_8$-$C_{16}$ alkyl trimethylammonium salts, $C_8$-$C_{16}$ alkyl di(hydroxyethyl)methylammonium salts, the $C_8$-$C_{16}$ alkyl hydroxyethyldimethylammonium salts, and $C_8$-$C_{16}$ alkyloxypropyltrimethylammonium salts. Of the above, decyl trimethylammonium methylsulfate, lauryl trimethylammonium chloride, myristyl trimethylammonium bromide and coconut trimethylammonium chloride and methylsulfate are particularly preferred.

A more complete disclosure of these and other cationic surfactants useful herein can be found in U.S. Patent 4,228,044, Cambre, issued October 14, 1980.

## Detergent Builders

Detergent compositions of the present invention contain inorganic and/or organic detergent builders to assist in mineral hardness control. These builders comprise from 0% to 80% by weight of the compositions. Built granular formulations preferably comprise from 10% to 80%, preferably 24% to 80%, by weight of detergent builder.

Suitable detergent builders include crystalline aluminosilicate ion exchange materials having the formula:

$$Na_z[(AlO_2)_z(SiO_2)_y].xH_2O$$

wherein z and y are at least 6, the mole ratio of z to y is from 1.0 to 0.5; and x is from 10 to 264. Amorphous hydrated aluminosilicate materials useful herein have the empirical formula

$$M_z(zAlO_2 \bullet ySiO_2)$$

wherein M is sodium, potassium, ammonium or substituted ammonium, z is from 0.5 to 2; and y is 1; this material having a magnesium ion exchange capacity of at least 50 milligram equivalents of $CaCO_3$ hardness per gram of anhydrous aluminosilicate.

The aluminosilicate ion exchange builder materials are in hydrated form and contain from 10% to 28% of water by weight if crystalline, and potentially even higher amounts of water if amorphous. Highly preferred crystalline aluminosilicate ion exchange materials contain from 18% to 22% water in their crystal matrix. The preferred crystalline aluminosilicate ion exchange materials are further characterized by a particle size diameter of from 0.1 $\mu$m to 10 $\mu$m. Amorphous materials are often smaller, e.g., down to less than 0.01 $\mu$m. More preferred ion exchange materials have a particle size diameter of from 0.2 $\mu$m to 4 $\mu$m. The crystalline aluminosilicate ion exchange materials are usually further characterized by their calcium ion exchange capacity, which is at least 200 mg. equivalent of $CaCO_3$ water hardness/g. of aluminosilicate, calculated on an anhydrous basis, and which generally is in the range of from 300 mg. eq./g. to 352 mg. eq./g. The aluminosilicate ion exchange materials are still further characterized by their calcium ion exchange rate which is at least 7.78 $gCa^{++}/\ell/s/g/\ell$ (2 grains $Ca^{++}$/gallon/minute/gram/gallon) of aluminosilicate (anhydrous basis), and generally lies within the range of from 7.78 $gCa^{++}/\ell/s/g/\ell$ (2 grains/gallon/minute/gram/gallon) to 23.33 $gCa^{++}/\ell/s/g/\ell$ (6 grains/gallon/minute/gram/gallon), based on calcium ion hardness. Optimum aluminosilicates for builder purposes exhibit a calcium ion exchange rate of at least 15.55 $gCa^{++}/\ell/s/g/\ell$ (4 grains/gallon/minute/gram/gallon).

The amorphous aluminosilicate ion exchange materials usually have a $Mg^{++}$ exchange capacity of at least 50 mg. eq. $CaCO_3$/g. (12 mg. $Mg^{++}$/g.) and a $Mg^{++}$ exchange rate of at least 3.89 $gCa^{++}/\ell/s/g/\ell$ (1 grain/gallon/minute/gram/gallon). Amorphous materials do not exhibit an observable diffraction pattern when examined by Cu radiation (1.54 Angstrom Units).

Useful aluminosilicate ion exchange materials are commercially available. These aluminosilicates can be crystalline or amorphous in structure and can be naturally-occurring aluminosilicates or synthetically derived. A method for producing aluminosilicate ion exchange materials is disclosed in U.S. Patent 3,985,669, Krummel, et al., issued October 12, 1976. Preferred synthetic crystalline aluminosilicate ion exchange materials useful herein are available under the designations Zeolite A, Zeolite P (B), and Zeolite X. In an especially preferred embodiment, the crystalline aluminosilicate ion exchange material has the formula

$Na_{12}[(AlO_2)_{12}(SiO_2)_{12}].xH_2O$

wherein x is from 20 to 30, especially about 27.

Other detergency builders useful in the present invention include the alkali metal silicates, alkali metal carbonates, phosphates, polyphosphates, phosphonates, polyphosphonic acids, $C_{10-18}$ alkyl monocarboxylic acids, polycarboxylic acids, alkali metal, ammonium or substituted ammonium salts thereof and mixtures thereof. The most preferred builders for use in the present invention are the alkali metal, especially sodium, salts of these compounds.

Specific examples of inorganic phosphate builders are sodium and potassium tripolyphosphate, pyrophosphate, polymeric metaphate having a degree of polymerization of from 6 to 21, and orthophosphate. Examples of polyphosphonate builders are the sodium and potassium salts of ethylene-1,1-diphosphonic acid, the sodium and potassium salts of ethane 1-hydroxy-1,1-diphosphonic acid and the sodium and potassium salts of ethane-1,1,2-triphosphonic acid. Other suitable phosphorus builder compounds are disclosed in U.S. Patent 3,159,581, Diehl, issued December 1, 1964; U.S. Patent 3,213,030, Diehl, issued October 19, 1965; U.S. Patent 3,400,148, Quimby, issued September 3, 1968; U.S. Patent 3,400,176, Quimby, issued September 3, 1968; U.S. Patent 3,422,021, Roy, issued January 14, 1969; and U.S. Patent 3,422,137, Quimby, issued September 3, 1968.

Examples of nonphosphorus, inorganic builders are sodium and potassium carbonate, bicarbonate, sesquicarbonate, tetraborate decahydrate, and silicate having a mole ratio of $SiO_2$ to alkali metal oxide of from 0.5 to 4.0, preferably from 1.0 to 2.4.

Useful water-soluble, nonphosphorus organic builders include the various alkali metal, ammonium and substituted ammonium polyacetates, carboxylates, polycarboxylates and polyhydroxysulfonates. Examples of polyacetate and polycarboxylate builders are the sodium, potassium, lithium, ammonium and substituted ammonium salts of ethylenediamine tetraacetic acid, nitrilotriacetic acid, oxydisuccinic acid, mellitic acid, benzene polycarboxylic acids, and citric acid.

Highly preferred polycarboxylate builders are disclosed in U.S. Patent 3,308,067, Diehl, issued March 7, 1967. Such materials include the water-soluble salts of homo- and copolymers of aliphatic carboxylic acids such as maleic acid, itaconic acid, mesaconic acid, fumaric acid, aconitic acid, citraconic acid and methylenemalonic acid.

Other builders include the carboxylated carbohydrates disclosed in U.S. Patent 3,723,322, Diehl, issued March 28, 1973.

A class of useful phosphorus-free detergent builder materials have been found to be ether polycarboxylates. A number of ether polycarboxylates have been disclosed for use as detergent builders. Examples of useful ether polycarboxylates include oxydisuccinate, as disclosed in Berg, U.S. Patent 3,128,287, issued April 7, 1964, and Lamberti et al, U.S. Patent 3,635,830, issued January 18, 1972.

A specific type of ether polycarboxylates useful as builders in the present invention are those having the general formula:

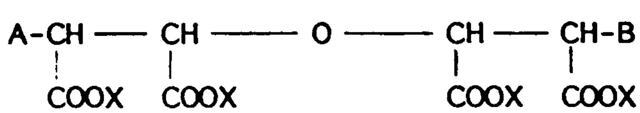

wherein A is H or OH; B is H or

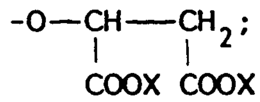

and X is H or a salt-forming cation. For example, if in the above general formula A and B are both H, then the compound is oxydissuccinic acid and its water-soluble salts. If A is OH and B is H, then the compound is tartrate monosuccinic acid (TMS) and its water-soluble salts. If A is H and B is

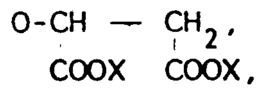

then the compound is tartrate disuccinic acid (TDS) and its water-soluble salts. Mixtures of these builders are especially preferred for use herein. Particularly preferred are mixtures of TMS and TDS in a weight ratio of TMS to TDS of from 97:3 to 20:80. These builders are disclosed in U.S. Patent 4,663,071 to Bush et al., issued May 5, 1987.

Suitable ether polycarboxylates also include cyclic compounds, particularly alicyclic compounds, such as those described in U.S. Patents 3,923,679; 3,835,163; 4,158,635; 4,120,874 and 4,102,903.

Other useful detergency builders include the ether hydroxypolycarboxylates represented by the structure:

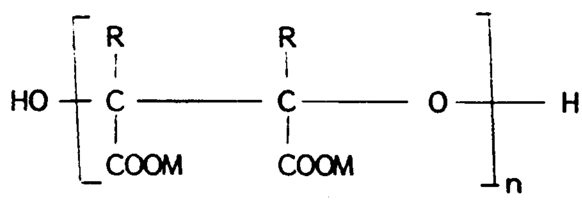

wherein M is hydrogen or a cation wherein the resultant salt is water-soluble, preferably an alkali metal, ammonium or substituted ammonium cation, n is from 2 to 15 (preferably n is from 2 to 10, more preferably n averages from 2 to 4) and each R is the same or different and selected from hydrogen, $C_{1-4}$ alkyl or $C_{1-4}$ substituted alkyl (preferably R is hydrogen).

Also suitable in the detergent compositions of the present invention are the 3,3-dicarboxy-4-oxa-1,6-hexanedioates and the related compounds disclosed in U.S. Patent 4,566,984, Bush, issued January 28, 1986. Other useful builders include the $C_5$-$C_{20}$ alkyl succinic acids and salts thereof. A particularly preferred compound of this type is dodecenylsuccinic acid.

Useful builders also include sodium and potassium carboxymethyloxymalonate, carboxymethyloxysuccinate, cis-cyclohexanehexacarboxylate, cis-cyclopentanetetracarboxylate phloroglucinol trisulfonate, water-soluble polyacrylates (having molecular weights of from 2,000 to 200,000, for example), and the copolymers of maleic anhydride with vinyl methyl ether or ethylene.

Other suitable polycarboxylates are the polyacetal carboxylates disclosed in U.S. Patent 4,144,226, Crutchfield et al., issued March 13, 1979. These polyacetal carboxylates can be prepared by bringing together, under polymerization conditions, an ester of glyoxylic acid and a polymerization initiator. The resulting polyacetal carboxylate ester is then attached to chemically stable end groups to stabilize the polyacetal carboxylate against rapid depolymerization in alkaline solution, converted to the corresponding salt, and added to a surfactant.

Especially useful detergency builders include the $C_{10}$-$C_{18}$ alkyl monocarboxylic (fatty) acids and salts thereof. These fatty acids can be derived from animal and vegetable fats and oils, such as tallow, coconut oil and palm oil. Suitable saturated fatty acids can also be synthetically prepared (e.g., via the oxidation of petroleum or by hydrogenation of carbon monoxide via the Fisher-Tropsch process). Particularly preferred $C_{10}$-$C_{18}$ alkyl monocarboxylic acids are saturated coconut fatty acids, palm kernel fatty acids, and mixtures thereof.

Other useful detergency builder materials are the "seeded builder" compositions disclosed in Belgian Patent 798,856, published October 29, 1973. Specific examples of such seeded builder mixtures are 3:1 wt.

mixtures of sodium carbonate and calcium carbonate having 5 micron particle diameter; 2.7:1 wt. mixtures of sodium sesquicarbonate and calcium carbonate having a particle diameter of 0.5 µm; 20:1 wt. mixtures of sodium sesquicarbonate and calcium hydroxide having a particle diameter of 0.01 µm; and a 3:3:1 wt. mixture of sodium carbonate, sodium aluminate and calcium oxide having a particle diameter of 5 µm.

## Chelating Agents

The detergent compositions herein may also optionally contain one or more iron and manganese chelating agents. Such chelating agents can be selected from the group consisting of amino carboxylates, amino phosphonates, polyfunctionally - substituted aromatic chelating agents and mixtures thereof, all as hereinafter defined. Without relying on theory, it is speculated that the benefit of these materials is due in part to their exceptional ability to remove iron and manganese ions from washing solutions by formation of soluble chelates.

Amino carboxylates useful as optional chelating agents in compositions of the invention have one or more, preferably at least two, units of the substructure

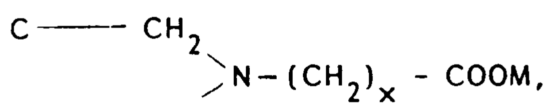

wherein M is hydrogen, alkali metal, ammonium or substituted ammonium (e.g. ethanolamine) and x is from 1 to 3, preferably 1. Preferably, these amino carboxylates do not contain alkyl or alkenyl groups with more than 6 carbon atoms. Alkylene groups can be shared by substructures. Operable amine carboxylates include ethylenediaminetetraacetates, N-hydroxyethylethylenediaminetriacetates, nitrilotriacetates, ethylenediamine tetraproprionates, triethylenetetraaminehexaacetates, diethylenetriaminepentaacetates, and ethanoldiglycines or mixtures thereof.

Amino phosphonates are also suitable for use as chelating agents in the compositions of the invention when at least low levels of total phosphorus are permitted in detergent compositions. Compounds with one or more, preferably at least two, units of the substructure

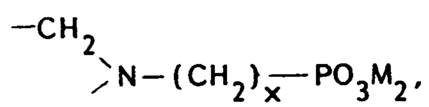

wherein M is hydrogen, alkali metal, ammonium or substituted ammonium and x is from 1 to 3, preferably 1, are useful and include ethylenediaminetetrakis (methylenephosphonates), nitrilotris (methylenephosphonates) and diethylenetriaminepentakis (methylenephosphonates). Preferably, these amino phosphonates do not contain alkyl or alkenyl groups with more than 6 carbon atoms. Alkylene groups can be shared by substructures.

Polyfunctionally - substituted aromatic chelating agents are also useful in the compositions herein. These materials comprise compounds having the general formula

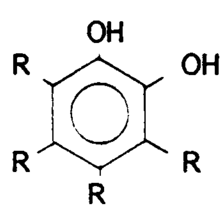

wherein at least one R is $-SO_3H$ or -COOH or soluble salts thereof and mixtures thereof. U.S. Patent 3,812,044 issued May 21, 1974, to Connor et al., discloses polyfunctionally - substituted aromatic chelating and sequestering agents. Preferred compounds of this type in acid form are dihydroxydisulfobenzenes and 1,2-dihydroxy -3,5-disulfobenzene or other disulfonated catechols in particular. Alkaline detergent compositions can contain these materials in the form of alkali metal, ammonium or substituted ammonium (e.g. mono-or triethanol-amine) salts.

If utilized, these chelating agents will generally comprise from 0.1% to 10% by weight of the detergent or laundry additive compositions herein. More preferably chelating agents will comprise from 0.75% to 3.0% by weight of such compositions.

## Bleaching Agents

The detergent compositions of the present invention can optionally contain from 1% to 20%, preferably 1% to 10% of percarboxylic acids bleaching agents or bleaching compositions containing peroxygen bleaches capable of yielding hydrogen peroxide in an aqueous solution and specific bleach activators, hereinafter defined, at specific molar ratios of hydrogen peroxide to bleach activator. These bleaching agents are fully described in U.S. Patent 4,412,934, Chung et al., issued November 1, 1983, and in U.S. Patent 4,483,781, Hartman, issued November 20, 1984. Such compositions provide extremely effective and efficient surface bleaching of textiles which thereby remove stains and/or soils from the textiles. The compositions are particularly effective at removing dingy soils from textiles. Dingy soils are soils that build up on textiles after numerous cycles of usage and washing and, thus, result in a white textile having a gray tint. These soils tend to be a blend of particulate and greasy materials. The removal of this type of soil is sometimes referred to as "dingy fabric clean up".

The bleaching compositions provide such bleaching over a wide range of bleach solution temperatures. Such bleaching is obtained in bleach solutions wherein the solution temperature is at least 5°C. Without the bleach activator such peroxygen bleaches would be ineffective and/or impracticable at temperatures below 60°C.

## The Peroxygen Bleaching Compound

The peroxygen bleaching compounds useful herein are those capable of yielding hydrogen peroxide in an aqueous solution. These compounds are well known in the art and include hydrogen peroxide and the alkali metal peroxides, organic peroxide bleaching compounds such as urea peroxide, and inorganic persalt bleaching compounds, such as the alkali metal perborates, percarbonates, perphosphates, and the like. Mixtures of two or more such bleaching compounds can also be used, if desired.

Preferred peroxygen bleaching compounds include sodium perborate, commercially available in the form of mono- and tetra-hydrate, sodium carbonate peroxyhydrate, sodium pyrophosphate peroxyhydrate, urea peroxyhydrate, and sodium peroxide. Particularly preferred are sodium perborate tetrahydrate and, especially, sodium perborate monohydrate. Sodium perborate monohydrate is especially preferred because it is very stable during storage and yet still dissolves very quickly in the bleaching solution.

Bleaching agents useful herein contain from 0.1% to 99.9% and preferably from 1% to 60% of these peroxygen bleaches.

## The Bleach Activator

The bleach activators within the invention have the general formula:

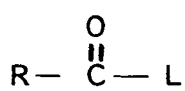

wherein R is an alkyl group containing from 1 to 18 carbon atoms wherein the longest linear alkyl chain extending from and including the carbonyl carbon contains from 6 to 10 carbon atoms and L is a leaving group, the conjugate acid of which has a $pK_a$ in the range of from 4 to 13.

L can be essentially any suitable leaving group. A leaving group is any group that is displaced from the bleach activator as a consequence of the nucleophilic attack on the bleach activator by the perhydroxide anion. This, the perhydrolysis reaction, results in the formation of the percarboxylic acid. Generally, for a group to be a suitable leaving group it must exert an electron attracting effect. This facilitates the nucleophilic attack by the perhydroxide anion. Leaving groups that exhibit such behavior are those in which their conjugate acid has a $pK_a$ in the range of from 4 to 13, preferably from 7 to 11 and most preferably from 8 to 11.

Preferred bleach activators are those of the above general formula wherein R is as defined in the general formula and L is selected from the group consisting of:

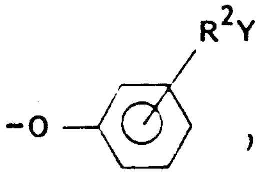 , 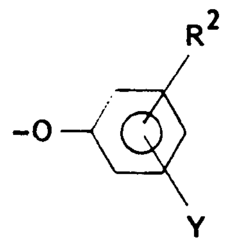 , 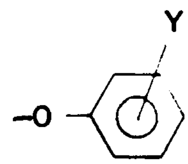 ,

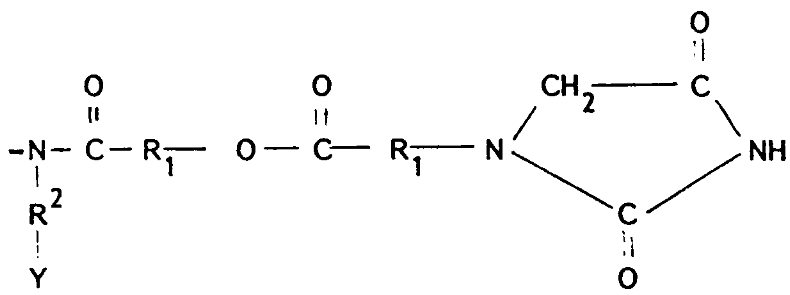 ,

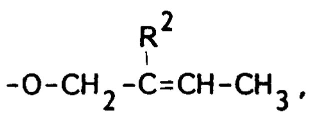 , 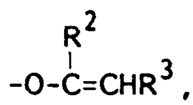 , 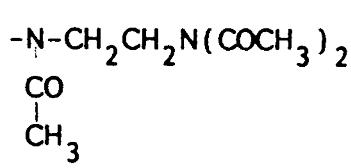

wherein R is as defined above, $R^2$ is an alkyl chain containing from 1 to 8 carbon atoms, $R^3$ is H or $R^2$, and Y is H or a solubilizing group. The preferred solubilizing groups are $-SO^-{}_3M^+$, $-COO^-M^+$, $-SO^-{}_4M^+$, $(-N^+R_3{}^4)X^-$ and $-O-NR_2{}^4$ and most preferably $-SO^-{}_3M^+$ and $-COO^-M^{+3}$ wherein $R^4$ is an alkyl chain containing from 1 to 4 carbon atoms, M is a cation which provides solubility to the bleach activator, and X is an anion which provides solubility to the bleach activator. Preferably, M is an alkali metal, ammonium or substituted ammonium cation, with sodium and potassium being most preferred, and X is a halide, hydroxide, methylsulfate or acetate anion. It should be noted that bleach activators with a leaving group that does not contain a solubilizing group should be well dispersed in the bleaching solution in order to assist in their dissolution.

Preferred bleach activators are also those of the above general formula wherein L is as defined in the general formula and R is an alkyl group containing from 1 to 12 carbon atoms wherein the longest linear alkyl chain extending from and including the carbonyl carbon contains from 6 to 10 carbon atoms.

Even more preferred are bleach activators of the above general formula wherein L is as defined in the general formula and R is a linear alkyl chain containing from 1 to 9 and preferably from 1 to 8 carbon atoms.

More preferred bleach activators are those of the above general formula wherein R is a linear alkyl chain containing from 5 to 9 and preferably from 6 to 8 carbon atoms and L is selected from the group consisting of:

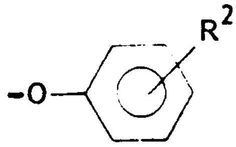,
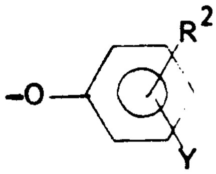,
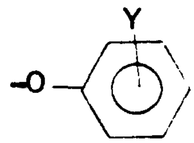,

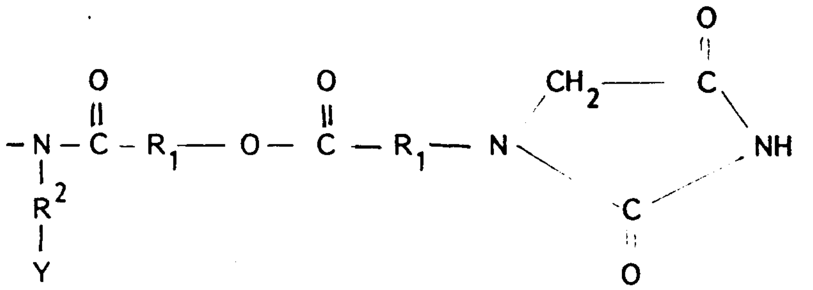,

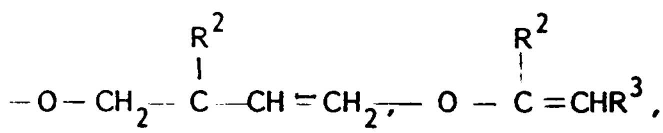,

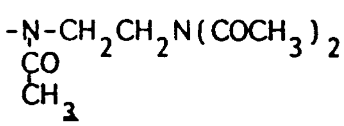

wherein R, $R^2$, $R^3$ and Y are as defined above.

Particularly preferred bleach activators are those of the above general formula wherein R is an alkyl group containing from 1 to 12 carbon atoms wherein the longest linear portion of the alkyl chain extending from and including the carbonyl carbon is from 1 to 10 carbon atoms and L is selected from the group consisting of:

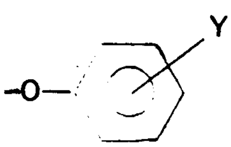,
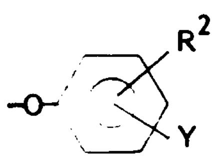,
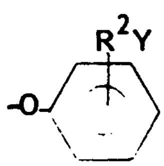 and

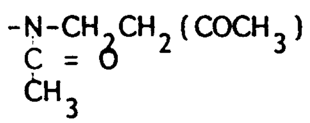

wherein $R^2$ is as defined above and Y is $-SO^-_3M^+$ or $-COO^-M^+$ wherein M is as defined above. A particularly preferred bleach activator from the above group is tetraacetyl ethylene diamine which is disclosed in European Patent Application 204,116, Hardy et al., published December 10, 1986.

Especially preferred bleach activators are those of the above general formula wherein R is a linear alkyl chain containing from 5 to 9 and preferably from 6 to 8 carbon atoms and L is selected from the group consisting of:

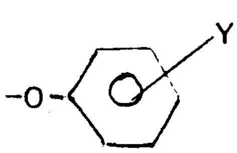,
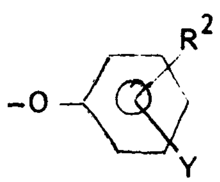
and
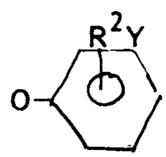

wherein $R^2$ is as defined above and Y is $-SO^-_3M^+$ or $-COO^-M^+$ wherein M is as defined above.

The more preferred bleach activators have the formula:

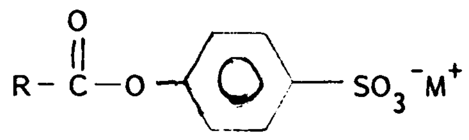

wherein R is a linear or branched alkyl chain containing from 5 to 9 and preferably from 6 to 8 carbon atoms and M is sodium or potassium. The most preferred bleach activator is sodium nonyl oxybenzene sulfonate. Sodium nonyloxbenzene sulfonate can also be used in combination with any of the above-described bleach activators, particularly tetraacetyl ethylene diamine.

These bleach activators can also be combined with up to 15% of binder materials (relative to the activator) such as nonionic surfactants, polyethylene glycols, fatty acids, anionic surfactants and mixtures thereof. Such binding materials are fully set forth in U.S. Patent 4,486,327, Murphy et al., issued December 4, 1984.

Bleaching agents useful herein contain from 0.1% to 60% and preferably from 0.5% to 40% of these bleach activators.

## Percarboxylic Acid Bleaching Agents

Bleaching agents can also comprise percarboxylic acids and salts thereof. Suitable examples of this class of agents include magnesium monoperoxyphthalate hexahydrate, the magnesium salt of meta-chloro perbenzoic acid, nonyl amino-6-oxoperoxysuccinic acid and diperoxydodecanedioic acid. Such bleaching agents are disclosed in U.S. Patent 4,483,781, Hartman, issued November 20, 1984, U.S. Patent Application 740,446, Burns et al., filed June 3, 1985 and also in European Patent Application 0,133,354, Banks et al., published February 20, 1985.

## Smectite Clay Minerals

A highly preferred optional component of formulations in accordance with the present invention is a smectite clay, which serves to provide additonal fabric softening performance. The smectite clays particularly useful in the present invention are montmorillonites, saponites, and hectorites. The clays used herein have particle size which cannot be perceived tactilely. Impalpable clays have particle sizes below about 50 microns.

The clay minerals used to provide fabric conditioning properties in the instant composition can be described as expandable (swellable), three-layer clays, in which a sheet of aluminum/oxygen atoms or magnesium/oxygen atoms lies between two layers of silicone/oxygen atoms, i.e., aluminosilicates and magnesium silicates, having an ion exchange capacity of at least 50 meq/100 g. of clay, and preferably at least 60 meq/100 g. of clay. The term "expandable" as used to describe clays relates to the ability of the layered clay structure to be swollen or expanded on contact with water. The three-layer expandable clays used herein are examples of the clay minerals classified geologically as smectites. Such smectite clays are described in Grim, Clay Mineralogy (2nd. Ed.) pp. 77-79 (1968), and in Van Olphen, An Introduction to Clay Colloid Chemistry, (2nd. Ed.) pp 64-76 (1977).

In general, there are two distinct classes of smectite clays that can be broadly differentiated on the basis of the number of octahedral metal-oxygen arrangements in the central layer for a given number of silicone-oxygen atoms in the outer layers. The dioctahedral minerals are primarily trivalent metal ion-based clays and are comprised of the prototype pyrophyllite and the members montmorillonite $(OH)_4Si_{8-y}Al_y(Al_{4-x}Mg_x)O_{20}$, nontronite $(OH)_4Si_{8-y}Al_y(Al_{4-x}Fe_x)O_{20}$, and volchonskoite $(OH)_4Si_{8-y}Al_y(Al_{4-x}Cr_x)O_{20}$, where x has a value of from 0 to 4.0 and y has a value of from 0 to 2.0.

The trioctahedral minerals are primarily divalent metal ion based and comprise the prototype talc and the members hectorite $(OH)_4Si_{8-y}Al_y(Mg_{6-x}Li_x)O_{20}$, saponite $(OH)_4Si_{8-y}Al_y(Mg_{6-x}Al_x)O_{20}$, sauconite $(OH)_4Si_{8-y}Al_y(Zn_{6-x}Al_x)O_2$, and vermiculite $(OH)_4Si_{8-y}Al_y(Mg_{6-x}Fe_x)O_{20}$, wherein y has a value of 0 to 2.0 and x has a value of 0 to 6.0.

The smectite minerals that are believed to be the most beneficial in fabric care and therefore more preferred when incorporated into detergent compositions are montmorillonites, hectorites and saponites, i.e. minerals of the structure $(OH)_4Si_{8-y}Al_y(Al_{4-x}Mg_x)O_{20}$, $(OH)_4Si_{8-y}Al_y(Mg_{6-x}Li_x)O_{20}$ and $(OH)_4Si_{8-y}Al_yMg_{6-x}Al_xO_{20}$ respectively in which the counter ions are predominantly sodium, potassium or

lithium, more preferably sodium or lithium. Especially preferred are beneficated forms of such clays. Benefication of clay removes the various impurities such as quartz thereby providing enhanced fabric care performance. Beneficiation can take place by any of a number of methods known in the art. Such methods include a conversion of clay into a slip and then passing it through a fine sieve and also flocculating or precipitation of suspended clay particles by the addition of acids or other electro-negatively charged substances. These and other methods of beneficating clay are described in Grinshaw, The Chemistry and Physics of Clay, pp 525-27 (1971).

As noted hereinabove, the clay minerals employed in the compositions of the instant invention contain exchangeable cations including, but not limited to, protons, sodium ions, potassium ions, calcium ions, magnesium ions and lithium ions.

It is customary to distinguish between clays on the basis of one cation predominantly or exclusively adsorbed. For example, a sodium clay is one in which the adsorbed cation is predominantly sodium. As used herein, the term clay, such as a montmorillonite clay, includes all the various exchangeable cation variants of that clay, e.g. sodium montmorillonite, potassium montmorillonite, lithium montmorillonite, magnesium montmorillonite or calcium montmorillonite.

Such adsorbed cations can become involved in exchange reactions with cations present in aqueous solutions. A typical exchange reaction involving a preferred smectite clay (montmorillonite clay (is expressed by the following equation:

montmorillonite clay (Na) + $NH_4OH$ = montmorillonite clay($NH_4$) + NaOH.

Since, in the foregoing equilibrium reaction, one equivalent weight of ammonium ion replaces an equivalent weight of sodium, it is customary to measure cation exchange capacity (sometimes termed "base exchange capacity") in terms of milliequivalents per 100 g. of clay (meq/100 g.). The cation exchange capacity of clays can be measured in several ways, including by electro-dialysis, by exchange with ammonium ion followed by titration or by a methylene blue procedure, all of which are fully set forth in Grimshaw, The Chemistry and Physics of Clays, supra at 264-265. The cation exchange capacity of a clay mineral relates to such factors as the expandable properties of the clay and the charge of the clay, which, in turn, is determined at least in part by the lattice structure. The ion exchange capacity of clays varies widely in the range from 2 meq/100 g. for kaolinites to 150 meq/100 g., and greater, for certain smectite clays such as montmorillonites. Montmorillonites, hectorites and saponites all have exchange capacities greater than 50 meq/100 g. and are therefore useful in the present invention. Illite clays, although having a three layer structure, are of a nonexpanding lattice type and have an ion exchange capacity somewhere in the lower portion of the range, i.e., around 26 meq/100 g. for an average illite clay. Attapulgites, another class of clay minerals, have a spicular (i.e. needle-like) crystalline form with a low cation exchange capacity (25-30 meq/100 g.). Their structure is composed of chains of silica tetrahedrons linked together by octahedral groups of oxygens and hydroxyls containing Al and Mg atoms.

Bentonite is a rock type clay originating from volcanic ash and contains montmorillonite (one of the preferred smectite clays) as its principal clay component. The following table shows that materials commercially available under the name bentonite can have a wide range of cation exchange capacities.

| Bentonite | Supplier | Exchange Capacity (meq/100 g.) |
|---|---|---|
| Brock® | Georgia Kaolin Co. USA | 63 |
| Soft Clark® | Georgia Kaolin Co. USA | 84 |
| Bentolite® L | Georgia Kaolin Co. USA | 68 |
| Clarolite® T-60 | Georgia Kaolin Co. USA | 61 |
| Granulare Naturale Bianco® | Seven C. Milan Italy | 23 |
| Thixo-Jel® #4 | Georgia Kaolin Co. USA | 55 |
| Granular Naturale Normale® | Seven C. Milan Italy | 19 |
| Clarsol® FB 5 | Ceca Paris France | 12 |
| PDL® 1740 | Georgia Kaolin Co. USA | 26 |
| Versuchs Product FFI | Sud-Chemie Munich, Germany | 26 |

Some bentonite clays (i.e., those with cationic exchange capacity above 50 meq/100 q.) can be used in the detergent compositions of the present invention.

It has been determined that illite, attapulgite, and kaolinite clays, with their relatively low ion exchange capacities, are not useful in the instant compositions. However, the alkali metal montmorillonites, saponites, and hectorites and certain alkaline earth metal varieties of these minerals, such as sodium hectorite, lithium hectorite and potassium hectorite, do meet the Ion exchange capacity criteria set forth above and have been found to show useful fabric care benefits when incorporated in detergent compositions in accordance with the present invention.

Specific non-limiting examples of commercially-available smectite clay minerals which provide fabric care benefits when incorporated into the detergent compositions of the present invention include:

<u>Sodium Hectorite</u>
Bentone EW®
Veegum F®
Laponite SP®
<u>Sodium Montmorillonite</u>
Brock®
Volclay BC®
Gelwhite GP®
Ben-A-Gel®
<u>Sodium Saponite</u>
Barasym® NAS 100
<u>Calcium Montmorillonite</u>
Soft Clark®
Gelwhite L®
<u>Lithium Hectorite</u>
Barasym® LIH 200

It is to be recognized that such smectite minerals obtained under the foregoing tradenames can comprise mixtures of the various discrete mineral entities. Such mixtures of the smectite minerals are suitable for use herein.

Within the classes of montmorillonites, hectorite and saponite clay minerals having a cation exchange capacity of at least about 50 meq/100g., certain clays are preferred for fabric softening purposes. For example. Gelwhite® GP is an extremely white form of smectite clay and is therefore preferred when formulating white granular detergent compositions. Volclay® BC, which is a smectite clay mineral containing at least 3% of iron (expressed as $Fe_2O_3$) in the crystal lattice, and which has a very high ion exchange capacity, is one of the most efficient and effective clays for use in detergent softening composition. Imvite® K is also very satisfactory.

Appropriate clay minerals for use herein can be selected by virtue of the fact that smectites exhibit a true 14 x $10^{-10}$ m (14Å) x-ray diffraction pattern. This characteristic pattern, taken in combination with exchange capacity measurements performed in the manner noted above, provides a basis for selecting particular smectite-type minerals for use in the compositions disclosed herein.

The smectite clay materials useful in the present invention are hydrophilic in nature, i.e., they display swelling characteristics in aqueous media. Conversely they do not swell in nonaqueous or predominantly non-aqueous systems.

The clay-containing detergent compositions according to the invention contain up to 35%, preferably from 4% to 15%, especially from 4% to 12%, by weight of clay.

Enzymes are a preferred optional ingredient and are incorporated in an amount of from 0.025% to 2%, preferably from 0.05% to 1.5%. Preferred proteolytic enzymes should provide a proteolytic activity of at least 5 Anson units (about 1,000,000 Delft units) per liter, preferably from 15 to 70 Anson units per liter, most preferably from 20 to 40 Anson units per liter. A proteolytic activity of from 0.01 to 0.05 Anson units per gram of product is desirable. Other enzymes, including amylolytic enzymes, are also desirably included in the present compositions.

Suitable proteolytic enzymes include the many species known to be adapted for use in detergent compositions. Commercial enzyme preparations such as "Savinase"® and Alcalase"® sold by Novo Industries and "Maxatase"® sold by Gist-Brocades, Delft, The Netherlands, are suitable. Other preferred enzyme compositions include those commercially available under the tradenames SP-72 ("Esperase")® manufactured and sold by Novo Industries, A/S, Copenhagen, Denmark and "AZ-Protease"® manufactured and sold by Gist-Brocades, Delft, The Netherlands.

Suitable amylases include "Rapidase"® sold by Gist-Brocades and "Termamyl"® sold by Novo Industries.

A more complete disclosure of suitable enzymes can be found in U.S. Patent No. 4,101,457, Place et al., issued July 18, 1978, and in U.S. Patent 4,507,219, Hughes, issued March 26, 1985.

## Other Optional Detergent Ingredients

Other optional ingredients which can be included in detergent compositions of the present invention, in their conventional art-established levels for use (generally from 0 to 20%), include solvents, hydrotropes, solubilizing agents, suds suppressors, processing aids, soil-suspending agents, corrosion inhibitors, dyes, fillers, optical brighteners, germicides, pH-adjusting agents (monoethanolamine, sodium carbonate, sodium hydroxide, etc.), enzymes, enzyme-stabilizing agents, perfumes, non-peroxy bleaches and bleach stabilizers.

Materials that provide clay soil removal/anti-redeposition benefits can also be incorporated in the detergent compositions of the invention. These clay soil removal/anti-deposition agents are usually included at from 0.1 to 10% by weight of the composition.

One group of preferred clay soil removal/anti-redeposition agents are the ethoxylated amines disclosed in European Patent Application 112,593, Vander Meer, published July 4, 1984. Another group of preferred clay soil removal/anti-redeposition agents are the cationic compounds disclosed in European Patent Application 111,965, Oh and Gosselink, published June 27, 1984. Other clay soil removal/anti-redeposition agents which can be used include the ethoxylated amine polymers disclosed in European Patent Application 111,984, Gosselink, published June 27, 1984; the zwitterionic compounds disclosed in European Patent Application 111,976, Rubingh and Gosselink, published June 27, 1984; the zwitterionic polymers disclosed in European Patent Application 112,592, Gosselink, published July 4, 1984; and the amine oxides disclosed in U.S. Patent 4,548,744, Connor, issued October 22, 1985.

Soil release agents such as those disclosed in the art to reduce oily staining of polyester fabrics, may also be used in the compositions of the present invention. U.S. Patent 3,962,152, issued June 8, 1976, Nicol et al., discloses copolymers of ethylene terephthalate and polyethylene oxide terephthalate as soil release agents. U.S. Patent 4,174,305, issued November 13, 1979, Burns et al., discloses cellulose ether soil release agents.

## Detergent Formulations

Granular detergent compositions embodying the present invention can be formed by conventional techniques, i.e., by slurrying the individual components (with the exception of the imidazoline) in water and then atomizing and spray-drying the resultant mixture, or by pan or drum agglomeration of the ingredients. The imidazoline particles can be added directly or are preferably agglomerated as described above and admixed into the composition.

The detergent compositions of the invention are particularly suitable for laundry use, but are also suitable for the cleaning of hard surfaces and for dishwashing.

In a laundry method aspect of the invention, typical laundry wash water solutions comprise from 0.1% to 2% by weight of the detergent compositions of the invention. Fabrics to be laundered are agitated in these solutions to effect cleaning, stain removal, and fabric care benefits. The pH of a 0.1% by weight aqueous solution of this composition will be in the range of from 7.0 to 11.0, preferably from 8.0 to 11.0, and most preferably from 9.0 to 10.5.

All parts, percentages and ratios herein are by weight unless otherwise specified.

## EXAMPLES

The following examples illustrate the present invention. The abbreviations used are:

| Code | Ingredient |
|---|---|
| $C_{13}$ LAS | sodium $C_{13}$ linear alkylbenzene sulfonate |
| $C_{45}$ AS | sodium $C_{14-15}$ alkyl sulfate |
| $C_{12}$ ABS | sodium $C_{12}$ alkyl benzene sulfonate |

| | |
|---|---|
| NI | $C_{12-13}$ alkyl polyethoxylate 6.5T T = stripped of lower ethoxylated fractions and fatty alcohol |
| STPP | sodium tripolyphosphate (contains 4% pyrophosphate) |
| silicate | sodium silicate (1.6r) |
| carbonate | $Na_2CO_3$ |
| DTPA | sodium diethylene triamine pentaacetate |
| sulfate | sodium sulfate |
| PB1 | sodium perborate monohydrate |
| OBS | sodium nonyl oxybenzene sulfonate |
| Enzyme | Alcalase® |
| Imidazoline - $C_{13}$LAS (X:Y) | hydrogenated tallow amidoethyl-2-hydrogenated tallow imidazoline - linear $C_{13}$ alkyl benzene sulfonate ion-pair complex, X:Y indicates molar ratio of imidazoline:alkyl benzene sulfonate |
| clay | sodium montmorillonite |
| Misc | can include optical brightener, suds suppressor, dispersant, and anti-redeposition agents. |

EXAMPLE I

A granular laundry detergent composition of the present invention is made as follows:

The following components are combined and then spray dried in a conventional manner to form detergent premix.

| | Parts by Weight |
|---|---|
| LAS | 10.8% |
| AS | 10.8% |
| STPP | 44.2% |
| NI | 1.7% |
| DTPA | 1.8% |
| Silicate | 16.8% |
| Minors and misc. ingredients | 3.4% |
| Water | 10.5% |

The ion-pair complex is formed by combining a 4:1 molar ratio of hydrogenated tallow amido ethyl-2-hydrogenated tallow imidazoline (obtained from Sherex Chemical Corporation, Dublin, Ohio as Varisoft® 445 Imidazoline) and linear $C_{13}$ alkyl benzene sulfonic acid. The resulting mixture is heated to 85°C under a

nitrogen blanket with agitation in a beaker to give a homogeneous fluid. This mixture is then cooled, with stirring, down to room temperature. The resulting ion-pair complex is frozen by liquid nitrogen and then ground in a Oster® Blender Pulsematic Model 16 for about 30 seconds. The ground particles are then sieved sequentially through a Tyler® Screen 100 (150 µm) and then through a Tyler® Screen 250 (63 µm). The fraction which remains on the 250 Screen is retained. The average particle size of the fraction ranges from about 80 to 120 µm (as determined by, for example, a Malvern® 2600 particle size analyzer), and greater than 50% by weight of the particles fall within the range of about 20 to about 200 µm. 9.5 parts of these ion-pair complex particles are then added to 90.5 parts of the premix and the resulting detergent composition is thorougly mixed to assure even distribution. The resulting detergent composition exhibits excellent cleaning and excellent fabric care benefits such as softness and static control.

EXAMPLES II - VII

The following detergent compositions are representative of the present invention and are made as described above in Example I.

| | II | III | IV | V | VI | VII |
|---|---|---|---|---|---|---|
| $C_{13}$LAS | 9.8 | 7.0 | 9.8 | 9.8 | 9.4 | 9.8 |
| $C_{45}$AS | 9.8 | 7.0 | 9.8 | 9.8 | 9.4 | 9.8 |
| NI | 1.5 | 1.1 | 1.5 | 1.5 | 0.9 | 1.5 |
| STPP | 40.0 | 28.7 | 40.0 | 40.0 | - | 40.0 |
| Silicate | 15.2 | 10.9 | 15.2 | 15.2 | 1.7 | 15.2 |
| Carbonate | - | 20.6 | - | - | 4.7 | - |
| Aluminosilicate | - | - | - | - | 23.0 | - |
| DTPA | 1.6 | 1.1 | 1.6 | 1.6 | - | 1.6 |
| Sulfate | - | - | - | - | 33.3 | - |
| PB1 | - | - | - | - | - | - |
| OBS | - | - | - | - | - | - |
| Clay | - | - | - | - | - | - |
| Imidazoline - $C_{13}$LAS (4:1) | 9.5 | 7.6 | - | - | 5.8 | - |
| Imidazoline-$C_{13}$LAS (2:1) | - | - | 9.5 | - | - | - |
| Imidazoline-$C_{13}$LAS (2.9:1) | - | - | - | 9.5 | - | - |
| Imidazoline-$C_{13}$LAS (8:1) | - | - | - | - | - | 9.5 |
| Ion-pair Particle Size (µm) | 100 | 100 | 100 | 100 | 100 | 100 |

These compositions give excellent cleaning as well as excellent static control and softening benefits (without impairing cleaning).

EXAMPLES VIII - XII

The following detergent compositions are representative of the present invention and are made as described above in Example I.

| | VIII | IX | X | XI | XII |
|---|---|---|---|---|---|
| $C_{13}$LAS | 7.3 | 7.3 | 7.3 | 6.5 | 18.5 |
| $C_{45}$AS | 7.3 | 7.3 | 7.3 | 6.5 | - |
| NI | 1.1 | 1.1 | 1.1 | 1.0 | - |
| STPP | 29.7 | 29.7 | 29.7 | 26.7 | 33.9 |
| Silicate | 11.3 | 11.3 | 11.3 | 10.2 | - |
| Carbonate | 15.4 | 15.4 | 15.4 | 15.1 | - |
| DTPA | 1.2 | 1.2 | 1.2 | 1.1 | - |
| Sulfate | - | - | - | 0.9 | 26.0 |
| PB1 | 5.1 | 5.1 | 5.1 | 5.0 | - |
| OBS | 5.8 | 5.8 | 5.8 | 6.8 | - |
| Clay | - | - | - | 4.8 | 8.2 |
| Imidazoline-$C_{13}$LAS (4:1) | 5.9 | 5.9 | 5.9 | 6.0 | 8.2 |
| Ion-pair Particle Size (μm) | 100 | 31 | 116 | 141 | 50 |

These compositions give excellent cleaning as well as excellent static control and softening benefits (without impairing cleaning).

## Claims

1. A conditioning agent comprising an imidazoline and an anionic surfactant, characterized in that it comprises water-insoluble particles having an average diameter of from 10 to 300 μm, preferably from 10 to 250 μm, more preferably from 10 to 110 μm, comprising an imidazoline-anionic surfactant ion-pair complex with a ratio of imidazoline to anionic surfactant of from 10:1 to 1:1, preferably from 8:1 to 1:1, more preferably from 5:1 to 2:1, most preferably about 4:1, having the formula:

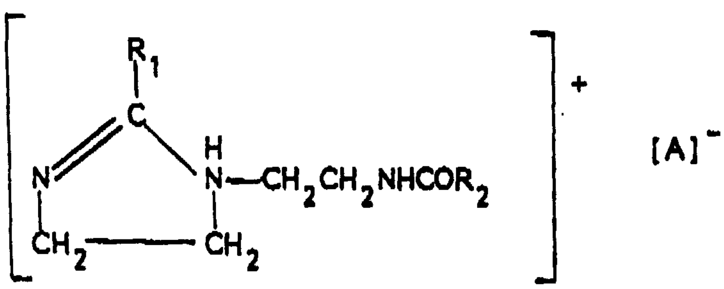

wherein each $R_1$ and $R_2$ can independently be $C_{12}$ to $C_{20}$ hydrocarbyl, preferably $C_{12}$ to $C_{20}$ alkyl or alkenyl, more preferably $C_{14}$ to $C_{20}$ alkyl, and A is aryl sulfonate, alkyl aryl sulfonate, paraffin sulfonate, olefin sulfonate, alkyl ethoxylated sulfate, dialkyl sulfosuccinate, ethoxylated alkyl sulfonate, alkyloxybenzene sulfonate, acyl isethionate, acylalkyl taurate, or a mixture of such ion-pair complexes, preferably linear $C_1$ to $C_{20}$ alkylaryl sulfonate, aryl sulfonate, dialkyl sulfosuccinate, or a mixture thereof.

2. A conditioning agent according to Claim 1, characterized in that the imidazoline component of said ion-pair complex is stearylamidoethyl-2-stearyl imidazoline, stearylamidoethyl-2-palmityl imidazoline, stearylamidoethyl-2-myristyl imidazoline, palmitylamidoethyl-2-palmityl imidazoline, palmitylamidoethyl-2-myristyl imidazoline, stearylamidoethyl-2-tallow imidazoline, myristylamidoethyl-2-tallow imidazoline, palmitylamidoethyl-2-tallow imidazoline, coconut-amidoethyl-2-coconut imidazoline, hydrogenated tallowamidoethyl-2-hydrogenated tallow imidazoline, or a mixture thereof, and preferably $R_1$ and $R_2$ are independently $C_{16}$ to $C_{18}$ alkyl.

3. A conditioning agent according to Claim 1, characterized in that it further comprises a smectite clay softening agent.

4. A detergent composition comprising:
   (a) from 1% to 98% by weight, preferably from 10% to 60% by weight, of a detergent surfactant selected from anionic surfactants, cationic surfactants, nonionic surfactants, zwitterionic surfactants,

amphoteric surfactants and mixtures thereof; and characterized by comprising
(b) from 0.1% to 25% by weight, preferably from 4% to 8% by weight, of the particles of Claim 1, 2, or 3;
wherein the pH of a 0.1% by weight aqueous solution of said composition is preferably in the range of from 7.0 to 11.0, more preferably from 9.0 to 10.5.

**5.** A detergent composition according to Claim 4, characterized in that the anionic surfactant component of said ion-pair complex is comprised of linear alkylbenzene sulfonates and alkyl sulfates, and the weight ratio of linear alkylbenzene sulfonate to alkyl sulfate is from 0.5:1 to 3:1.

**6.** A detergent composition according to Claim 4 or 5, characterized in that it additionally comprises from 10% to 80% by weight of detergency builder, said builder being a nitrilotriacetate or an inorganic phosphate, alkali metal, ammonium or unsubstituted ammonium salt, or a mixture thereof, wherein said composition optionally comprises: a) from 0.1% to 10% by weight, preferably from 0.75% to 3.0% by weight, of a chelating agent, said chelating agent preferably being an ethylenediaminetetraacetate, N-hydroxyethylethylenediaminetriacetate, nitrilotriacetate, ethylenediamine tetrapropionate, triethylenetetraaminehexaacetate, dimethylenetriaminepentaacetate,and ethanoidiglycine, alkali metal, ammonium or substituted ammonium salt, or a mixture thereof; b) from 1% to 20% by weight of inorganic or organic peroxy bleaching agent, said bleaching agent preferably comprising from 1.0% to 60% by weight of a peroxygen bleaching compound and from 0.5% to 40% by weight of a bleach activator; c) from 4% to 15% by weight, preferably 4% to 12% by weight, of a smectite clay, said clay preferably being sodium hectorite, potassium hectorite, lithium hectorite, magnesium hectorite, calcium hectorite, sodium montmorillonite, potassium montmorillonite, lithium montmorillonite, magnesium montmorillonite, calcium montmorillonite, sodium saponite, potassium saponite, lithium saponite, magnesium saponite, calcium saponite, or a mixture thereof; or d) from 0.025% to 2.0% by weight of a proteolytic enzyme.

**7.** A conditioning agent according to Claim 1, 2 or 3 characterized in that it comprises said particles in a liquid carrier.

**8.** A compact, through the wash laundry product, comprising:
a laminate of two plies of which at least one ply comprises a strong, high stretch tissue which is deeply embossed to form one or a multiplicity of nonconnecting cups surrounded by rims;
the tissue in each cup having been stretched 15% to 100% by said deep embossing;
said cups containing from 1% to 99% by weight of a conditioning agent; and optionally containing a laundry active, said active preferably being powdered detergent, builder, brightener, softener, enzyme, bleach solid, or a mixture thereof, and also optionally containing a smectite clay softening agent;
the other of said two plies covering the deeply embossed ply forming patterned cells which contain the powder, said plies being sealed on said rims to provide sold compact, through the wash laundry product;
said high stretch embossed tissue originally having a dry CD stretch of from 9% to 25% and a dry MD stretch of from 30% to 60%, a wet cross-directional tensile strength of from 78.7 to 315 g/cm, said one ply selected to withstand the stretching and said two plies selected to survive automatic washing and drying cycles without significant tearing; said tissue having sufficient porosity to permit the laundry actives to flow through the tissue, characterized in that said conditioning agent is that of Claim 1, 2, or 3.

**Patentansprüche**

**1.** Konditionierendes Mittel aus einem Imidazolin und einer anionischen grenzflächenaktiven Verbindung, dadurch gekennzeichnet, daß es wasserunlösliche Teilchen mit einem mittleren Durchmesser von 10 bis 300 µm, vorzugsweise von 10 bis 250 µm, stärker bevorzugt von 10 bis 110 µm, umfaßt, welche aus einem Ionenpaar-Komplex aus einem Imidazolin und einer anionischen grenzflächenaktiven Verbindung mit einem Verhältnis von Imidazolin zu der anionischen grenzflächenaktiven Verbindung von 10:1 bis 1:1, vorzugsweise von 8:1 bis 1:1, stärker bevorzugt von 5:1 bis 2:1, am stärksten bevorzugt etwa 4:1, welcher Komplex die Formel:

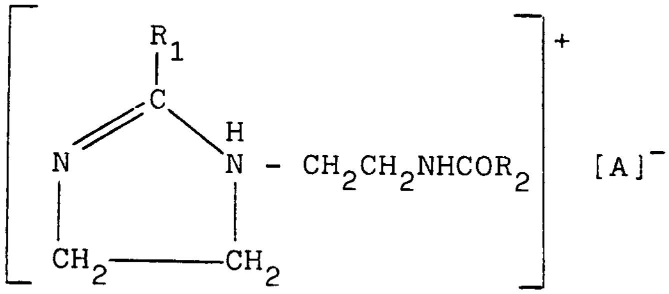

besitzt, worin jeder Rest $R_1$ und $R_2$ unabhängig voneinander $C_{12}$-$C_{20}$Hydrocarbyl, vorzugsweise $C_{12}$-$C_{20}$-Alkyl oder -Alkenyl, stärker bevorzugt $C_{14}$-$C_{20}$-Alkyl sein kann und A Arylsulfonat, Alkylarylsulfonat, Paraffinsulfonat, Olefinsulfonat, ethoxyliertes Alkylsulfat, Dialkylsulfosuccinat, ethoxyliertes Alkylsulfonat, Alkyloxybenzolsulfonat, Acylisethionat, Acylalkyltaurat, vorzugsweise lineares $C_1$-$C_{20}$-Alkylarylsulfonat, Arylsulfonat, Dialkylsulfosuccinat, oder ein Gemisch hievon bedeutet, oder aus einem Gemisch solcher Ionenpaar-Komplexe bestehen.

**2.** Konditionierendes Mittel nach Anspruch 1, dadurch gekennzeichnet, daß die Imidazolinkomponente des genannten Ionenpaar-Komplexes von Stearylamidoethyl-2-stearylimidazolin, Stearylamidoethyl-2-palmitylimidazolin, Stearylamidoethyl-2-myristylimidazolin, Palmitylamidoethyl-2-palmitylimidazolin, Palmitylamidoethyl-2-myristylimidazolin, Stearylamidoethyl-2-talgimidazolin, Myristylamidoethyl-2-talgimidazolin, Palmitylamidoethyl-2-talgimidazolin, Kokosnuß-amidoethyl-2-kokosnußimidazolin, hydrierter Talg-amidoethyl-2-(hydrierter Talg)-imidazolin oder einem Gemisch hievon gebildet wird, und die Reste $R_1$ und $R_2$ vorzugsweise unabhängig voneinander $C_{16}$-$C_{18}$-Alkyl darstellen.

**3.** Konditionierendes Mittel nach Anspruch 1, dadurch gekennzeichnet, daß es ferner einen Smectitton-Weichmacher enthält.

**4.** Detergenszusammensetzung, umfassend:

(a) 1 Gew.-% bis 98 Gew.-%, vorzugsweise 10 Gew.-% bis 60 Gew.-% eines grenzflächenaktiven Detergensmittels, welches unter anionischen grenzflächenaktiven Mitteln, kationischen grenzflächenaktiven Mitteln, nichtionischen grenzflächenaktiven Mitteln, zwitterionischen grenzflächenaktiven Mitteln, amphoteren grenzflächenaktiven Mitteln und Gemischen hievon ausgewählt ist;

und dadurch gekennzeichnet, daß sie

(b) 0,1 Gew.-% bis 25 Gew.-%, vorzugsweise 4 Gew.-% bis 8 Gew.-% der Teilchen nach Anspruch 1, 2 oder 3 umfaßt;

worin der pH-Wert einer 0,1 gew.-%igen wäßrigen Lösung der genannten Zusammensetzung vorzugsweise im Bereich von 7,0 bis 11,0, stärker bevorzugt von 9,0 bis 10,5 beträgt.

**5.** Detergenszusammensetzung nach Anspruch 4, dadurch gekennzeichnet, daß die anionische grenzflächenaktive Komponente des genannten Ionenpaar-Komplexes von linearen Alkylbenzolsulfonaten und Alkylsulfaten gebildet wird, und daß das Gewichtsverhältnis von linearem Alkylbenzolsulfonat zu Alkylsulfat von 0,5:1 bis 3:1 beträgt.

**6.** Detergenszusammensetzung nach Anspruch 4 oder 5, dadurch gekennzeichnet, daß sie zusätzlich 10 Gew.-% bis 80 Gew.-% an Detergensgerüststoff umfaßt, welcher Gerüststoff ein Nitrilotriacetat oder ein anorganisches Phosphat-, Alkalimetall-, Ammonium- oder unsubstituiertes Ammoniumsalz oder ein Gemisch hievon ist,

welche Zusammensetzung wahlweise:

a) 0,1 Gew.-% bis 10 Gew.-%, vorzugsweise 0,75 Gew.-% bis 3,0 Gew.-% eines Chelatbildners, welcher Chelatbildner vorzugsweise ein Ethylendiamintetraacetat, N-Hydroxyethylethylendiamintriacetat, Nitrilotriacetat, Ethylendiamintetrapropionat, Triethylentetraaminhexaacetat, Dimethylentriaminpentaacetat, und Ethanoldiglycin, ein Alkalimetall-, Ammonium- oder substituiertes Ammoniumsalz oder ein Gemisch hievon ist;

b) 1 Gew.-% bis 20 Gew.-% eines anorganischen oder organischen Peroxybleichmittels, welches Bleichmittel vorzugsweise 1,0 Gew.-% bis 60 Gew.-% einer Persauerstoffbleichmittelverbindung und

0,5 Gew.-% bis 40 Gew.-% eines Bleichmittelaktivators umfaßt;

c) 4 Gew.-% bis 15 Gew.-%, vorzugsweise 4 Gew.-% bis 12 Gew.-% eines Smectittones, welcher Ton vorzugsweise Natriumhectorit, Kaliumhectorit, Lithiumhectorit, Magnesiumhectorit, Calciumhectorit, Natriummontmorillonit, Kaliummontmorillonit, Lithiummontmorillonit, Magnesiummontmorillonit, Calciummontmorillonit, Natriumsaponit, Kaliumsaponit, Lithiumsaponit, Magnesiumsaponit, Calciumsaponit oder ein Gemisch hievon ist;

oder

d) 0,025 Gew.-% bis 2,0 Gew.-% eines proteolytischen Enzyms umfaßt.

**7.** Konditionierendes Mittel nach Anspruch 1, 2 oder 3, dadurch gekennzeichnet, daß es die genannten Teilchen in einem flüssigen Träger umfaßt.

**8.** Kompaktes, während des Waschens verwendbares Wäschewaschprodukt, umfassend:

ein Laminat aus zwei Lagen, wovon mindestens eine Lage ein festes, hoch-dehnbares Tissuepapier umfaßt, welches tiefgeprägt ist, um eine oder eine Vielzahl von nicht-verbundenen Vertiefungen auszubilden, welche von Stegen umgeben sind;

welches Tissuepapier in jeder Vertiefung beim Tiefprägen um 15% bis 100% gedehnt wurde;

welche Vertiefungen 1 Gew.-% bis 99 Gew.-% eines konditionierenden Mittels enthalten, und wahlweise wirksame Wäschewaschmaterialien beinhalten, welche wirksamen Materialien vorzugsweise pulverisierte Detergensmittel, Gerüststoffe, Aufheller, Weichmacher, Enzyme, Bleichmittelfeststoffe oder ein Gemisch hievon sind, und ebenfalls wahlweise einen Smectitton-Weichmacher enthalten;

worin die andere der beiden genannten Lagen die tiefgeprägte Lage bedeckt, um so musterförmig angeordnete Zellen auszubilden, welche das Pulver enthalten, welche Lagen an den Stegen miteinander verbunden sind, um das genannte kompakte, während des Waschens verwendbare Wäschewaschprodukt zu erhalten;

welches hoch-dehnbare, geprägte Tissuepapier ursprünglich eine Trockendehnung quer zur Maschinenrichtung von 9% bis 25% und eine Trockendehnung in Maschinenrichtung von 30% bis 60%, eine Naßzugfestigkeit quer zur Maschinenrichtung von 78,7 bis 315 g/cm aufweist, wobei die eine Lage ausgewählt ist, um die Dehnung auszuhalten, und die beiden Lagen ausgewählt sind, um die automatischen Wasch- und Trocknungskreisläufe ohne ein bedeutsames Reißen zu überstehen; welches Tissuepapier eine ausreichende Porosität besitzt, um es den wirksamen Wäschewaschmaterialien zu ermöglichen, durch das Tissuepapier zu gelangen,

dadurch gekennzeichnet, daß es sich bei dem genannten konditionierenden Mittel um eines nach den Ansprüchen 1, 2 oder 3 handelt.

## Revendications

**1.** Agent de conditionnement comprenant une imidazoline et un tensioactif anionique, caractérisé en ce qu'il comprend des particules insolubles dans l'eau ayant un diamètre moyen de 10 à 300 µm, de préférence de 10 à 250 µm, encore mieux de 10 à 110 µm, comprenant un complexe de couple d'ions imidazoline-tensioactif anionique avec un rapport de l'imidazoline au tensioactif anionique allant de 10:1 à 1:1, de préférence de 8:1 à 1:1, encore mieux de 5:1 à 2:1, au mieux d'environ 4:1, de formule:

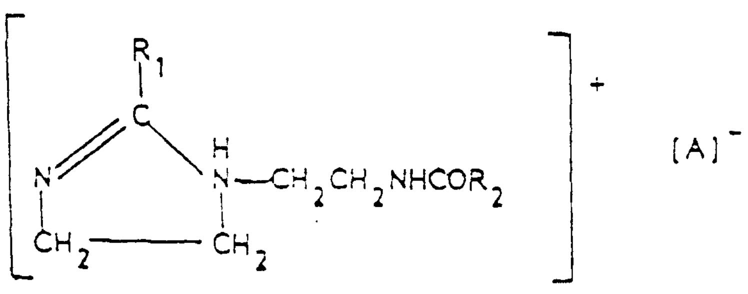

dans laquelle chacun des radicaux $R_1$ et $R_2$ peut être, indépendamment, un radical hydrocarboné en $C_{12}$-$C_{20}$, de préférence alkyle ou alcényle en $C_{12}$-$C_{20}$, encore mieux alkyle en $C_{14}$-$C_{20}$, et A est un groupe arylsulfonate, alkylarylsulfonate, paraffinesulfonate, oléfinesulfonate, alkylsulfate, oxyéthylé, dialkylsulfosuccinate, alkylsulfonate oxyéthylé, alkyloxybenzènesulfonate, acyliséthionate, acylakyltaurate,

ou un mélange de tels complexes de couples d'ions, de préférence un alkyl ($C_1$-$C_{20}$)-arylsulfonate, arylsulfonate, dialkylsulfosuccinate ou un mélange de ceux-ci.

**2.** Agent de conditionnement selon la revendication 1, caractérisé en ce que le composant imidazoline dudit complexe de couple d'ions est un composant stéarylamidoéthyl-2-stéarylimidazoline, stéarylamidoéthyl-2-palmitylimidazoline,stéaryl aminoéthyl-2-myristyl-imidazoline, palmitylamidoéthyl-2-palmityl-imidazoline, palmitylamidoéthyl-2-pyristyl-imidazoline, stéarylamidoéthyl-2-suif-imidazoline, myristylamidoéthyl-2-suif-imidazoline,palmitylamidoéthyl-2-suif-imidazoline, cocosamidoéthyl-2-coco-imidazoline, (suif hydrogéné)-amidoéthyl-2-suif hydrogéné-imidazoline ou un mélange de deux-ci, et de préférence $R_1$ et $R_2$ sont indépendamment un groupe alkyle en $C_{16}$-$C_{18}$.

**3.** Agent de conditionnement selon la revendication 1, caractérisé en ce qu'il comprend en outre un assouplissant de type argile smectique.

**4.** Composition de détergent comprenant:

(a) de 1 à 98% en poids, de préférence de 10 à 60% en poids d'un tensioactif détergent choisi parmi des tensioactifs anioniques, tensioactifs cationiques, tensioactifs non ioniques, tensioactifs zwittérioniques, tensioactifs amphotères et des mélanges de ceux-ci; et caractérisé en ce qu'elle comprend

(b) de 0,1 à 25% en poids, de préférence de 4 à 8% en poids, des particules de la revendication 1, 2 ou 3;

le pH d'une solution aqueuse à 0,1% en poids de ladite composition étant de préférence dans l'intervalle allant de 7,0 à 11,0, encore mieux de 9,0 à 10,5.

**5.** Composition de détergent selon la revendication 4, caractérisée en ce que le composant tensioactif anionique dudit complexe de couple d'ions est composé d'alkylbenzènesulfonates linéaires et d'alkylsulfates et le rapport pondéral de l'alkylbenzènesulfonate linéaire à l'alkylsulfate va de 0,5:1 à 3:1.

**6.** Composition de détergent selon la revendication 4 ou 5, caractérisée en ce qu'elle comprend en outre de 10 à 80% en poids d'adjuvant de détergence, ledit adjuvant étant un nitrilotriacétate ou un phosphate minéral, sel de métal alcalin, d'ammonium ou d'ammonium substitué, ou un mélange de ceux-ci, ladite composition comprenant éventuellement: a) de 0,1 à 10% en poids, de préférence de 0,75 à 3,0% en poids d'un agent chélateur, ledit agent chélateur étant de préférence un éthylènediaminetétraacétate, N-hydroxyéthylétylènediaminetriacétate, nitrilotriacétate, éthylènediaminetétrapropionate, triéthylènetétraminehexaacétate, diméthylènetriaminepentaacétate et l'éthanoldiglycine, sel de métal alcalin, d'ammonium ou d'ammonium substitué, ou un mélange de ceux-ci; b) de 1 à 20% en poids d'un agent de blanchiment de type peroxyde organique ou minéral, ledit agent de blanchiment comprenant de préférence de 1,0 à 60% en poids d'un agent de blanchiment de type peroxyde et de 0,5 à 40% en poids d'un activateur de blanchiment; c) de 4 à 15% en poids, de préférence de 4 à 12% en poids d'une argile smectique, ladite argile étant de préférence l'hectorite sodique, l'hectorite potassique, l'hectorite lithiée, l'hectorite magnésienne, l'hectorite calcique, la montmorillonite sodique, la montmorillonite potassique, la montmorillonite lithiée, la montmorillonite magnésienne, la montmorillonite calcique, la saponite sodique, la saponite potassique, la saponite lithiée, la saponite magnésienne, la saponite calcique ou un mélange de celles-ci; ou d) de 0,025 à 2,0% en poids d'un enzyme protéolytique.

**7.** Agent de conditionnement selon la revendication 1, 2 ou 3, caractérisé en ce qu'il comprend lesdites particules dans un support liquide.

**8.** Produit lessiviel compact, à utiliser en cycle, comprenant:

- un stratifié bicouche dont au moins une couche comprend un tissu résistant, très élastique, qui est profondément gaufré pour former une ou plusieurs cupules non reliées entourées par des rebords;
- le tissu dans chaque cupule ayant été étiré de 15 à 100% par ledit gaufrage profond;
- lesdites cupules contenant de 1 à 99% en poids d'un agent de conditionnement et contenant éventuellement un agent actif lessiviel, ledit agent actif étant de préférence un détergent en poudre, un adjuvant de détergence, un agent d'activage, un assouplissant, une enzyme, un agent de blanchiment solide ou un mélange de ceux-ci, et en outre contenant éventuellement un

assouplissant de type argile smectique;

- l'autre desdites deux couches couvrant la couche profondément gaufrée, formant des cellules disposées en motifs qui contiennent la poudre, lesdites couches étant scellées sur lesdits rebords pour donner ledit produit lessiviel compact, à utiliser en cycle;
- ledit tissu gaufré très élastique ayant initialement une élasticité transversale à l'état sec de 9 à 25% et une élasticité longitudinale à l'état sec de 30 à 60%, une résistance à la traction transversale à l'état humide de 78,7 à 315 g/cm, ladite couche étant choisie pour résister à l'étirage et lesdites deux couches étant choisies pour résister à des cycles de lavage et de séchage automatique sans déchirure importante, ledit tissu ayant une porosité suffisante pour permettre aux agents actifs lessiviels de passer à travers le tissu,

caractérisé en ce que ledit agent de conditionnement est celui de la revendication 1, 2 ou 3.